# EUROPÄISCHE PATENTSCHRIFT

(11) **EP 2 022 522 B1**
(45) Veröffentlichungstag und Bekanntmachung des Hinweises auf die Patenterteilung: **28.12.2011**
(21) Anmeldenummer: 07014560.2
(22) Anmeldetag: 25.07.2007
(51) Int. Cl.: A61M 5/32

(54) **Vorrichtung und Verfahren zur Injektion von Medien in menschliche und tierische Gewebe**
Devices and methods for the injection of agents into human and animal tissue
Dispositif et procédé destinés à l'injection de milieux dans des tissus humains et animaux

(43) Veröffentlichungstag der Anmeldung: 11.02.2009
(73) Patentinhaber: W & H Dentalwerk Bürmoos GmbH, 5111 Bürmoos (AT)
(72) Erfinder: Teufelberger, Gunther, 5111 Bürmoos (AT); Wagner, Hannes, 5023 Salzburg (AT); Oberascher, Christine, 5151 Nussdorf (DE); Kapeller, Stefan, 5151 Nussdorf (DE); Sigl, Rainer, 5120 Haigermoos (AT); Hörmann, Udo, 5411 Oberalm (DE)

(56) Entgegenhaltungen:
- WO-A-00/09186
- WO-A-00/67822
- WO-A-01/32241
- WO-A-96/30065
- WO-A-96/39213
- WO-A-2007/018809
- DE-A1- 10 254 442
- FR-A1- 2 581 548
- US-A- 2 773 500
- US-A- 3 811 442

## Beschreibung

Die vorliegende Erfindung betrifft Vorrichtungen und Verfahren zur Injektion von Medien in menschliche und tierische Gewebe, insbesondere in Hartgewebe.

Aus der Patentschrift US 4,787,893 ist ein Handgriffelement zur Injektion von flüssigen Substanzen, insbesondere von Anästhetika, bekannt, bei dem durch einen Antriebsmotor eine Aufnahmehülse in Rotation versetzt wird. In die Aufnahmehülse ist die Ampulle mit der zu injizierenden Substanz einfügbar, so dass die Drehbewegung auf die Ampulle und die damit verbundene Injektionsnadel übertragen wird. Das Handgriffelement wird somit nicht nur zur Injektion verwendet, sondern auch zum Ein- oder Durchdringen von Gewebe, zum Beispiel von Knochen, insbesondere des Kieferknochens, in das die Substanz injiziert werden soll.

Die Verbindung der Ampulle mit der Injektionsnadel erfolgt durch Durchstoßen eines Gummistopfens, des so genannten Septums, der an einem Ende der Ampulle vorgesehen ist, so dass die Injektionsnadel bis in das Ampullenreservoir mit der Injektionsflüssigkeit ragt. Diese Verbindung hat sich jedoch als nachteilig herausgestellt, da beim Durchbohren des Knochens die als Bohrer dienende Nadel gebremst wird, die Haltekraft des Gummistopfens für die Nadel jedoch zu gering ist, um die Bremswirkung auch auf die Ampulle zu übertragen, so dass es zu einer Relativbewegung zwischen der Nadel und der Ampulle kommt. Durch diese Relativbewegung, d.h. durch den schneller rotierenden Gummistopfen und die gebremste, langsamer rotierende Injektionsnadel, kommt es zu Abrieb am Gummistopfen. Zumindest ein Teil des Abriebs gelangt in Form feiner Gummipartikel in die Injektionsflüssigkeit, so dass die Gefahr besteht, dass diese Partikel mit der Injektionsflüssigkeit in das Gewebe injiziert werden. Ein weiterer Teil des Abriebs gelangt in das Handgriffelement und kann dort zu Beeinträchtigungen oder Schäden an Bauteilen führen.

Aus der Patentschrift US 3,811,442 ist ein Handgriffelement zur Injektion von Flüssigkeiten bekannt, das einen Griffabschnitt und einen Lagerabschnitt für eine Injektionsspritze, welche eine Ampulle und eine Nadel umfasst, aufweist. Am Lagerabschnitt sind zwei Lagerhülsen zur drehbaren Aufnahme der Ampulle und der Nadel vorgesehen. Eine Antriebseinheit versetzt die Injektionsspritze in Rotation, wobei die Drehmomentübertragung.auf die Ampulle erfolgt.

Die Patentschrift US 2,773,500 offenbart ein Handgriffelement zur Injektion von Flüssigkeiten mit einer Antriebseinheit, die eine Hohlwelle in Rotation versetzt. An jeweils ein Ende der Hohlwelle werden die Injektionsnadel und die Ampulle angeschlossen, so dass die Flüssigkeit aus der Ampulle durch die Hohlwelle und die Nadel an die Injektionsstelle gefördert wird.

Aus der Patentanmeldung WO 00/67822 A1 ist eine Injektionsspritze mit einer manuell zu bedienenden Fördervorrichtung bekannt, um die Injektionsflüssigkeit aus der Ampulle zu fördern. An der Injektionsspritze sind des Weiteren Mittel vorgesehen, zum Beispiel eine spiralförmige Nut, die bei Betätigen der Fördervorrichtung die Injektionsnadel und gegebenenfalls auch die Ampulle in Rotation versetzen.

Der vorliegenden Erfindung liegt daher die Aufgabe zugrunde ein Handgriffelement derart auszubilden, dass kein Abrieb entsteht.

Diese Aufgabe wird gemäß der vorliegenden Erfindung durch ein medizinisches Handgriffelement mit einer Kopplungseinrichtung zur drehfesten Kopplung oder Verbindung der Ampulle mit der Injektionsnadel gelöst. Die Kopplungseinrichtung bewirkt eine, bevorzugt mechanische, Kopplung der Ampulle mit der Injektionsnadel oder eine Verdrehsicherung zwischen der Ampulle und der Injektionsnadel, so dass die Ampulle und die Injektionsnadel mit der im Wesentlichen gleichen Drehzahl rotieren, wodurch eine Relativbewegung zwischen der Ampulle und der Injektionsnadel und damit der Abrieb des Gummistopfens (Septums) der Ampulle vermieden werden.

Damit die Ampulle und die Injektionsnadel mit der im Wesentlichen gleichen Drehzahl rotieren, ist sowohl eine zuverlässige Übertragung des Drehmoments von der Antriebsvorrichtung des medizinischen Handgriffelements auf die Ampulle und die Injektionsnadel, als auch eine Weiterleitung einer zum Beispiel beim Durchbohren von Knochengewebe auftretenden Bremskraft auf die Injektionsnadel und die Ampulle notwendig. Die Kopplungseinrichtung weist dafür eine Kupplungsvorrichtung mit zumindest einem Kupplungselement auf das insbesondere als Federlaschen, Federarm, Spannflächen, Gewinde, Mitnehmer oder Formschlusselemente ausgebildet ist, oder ist mit einer derartigen Kupplungsvorrichtung verbunden. Das zumindest eine Kupplungselement kuppelt die Ampulle und / oder die Injektionsnadel direkt oder indirekt an die Antriebsvorrichtung und bevorzugt auch an die Kopplungseinrichtung, so dass auf die Ampulle, die Injektionsnadel und die Kopplungseinrichtung im Wesentlichen das gleiche Drehmoment einwirken.

Eine weiteres Erfordernis für den Antrieb der Ampulle und der Injektionsnadel mit der im Wesentlichen gleichen Drehzahl besteht in einer zuverlässigen Verbindung der Ampulle mit der Injektionsnadel. Die Kopplungseinrichtung weist dafür eine Anschlussvorrichtung für die Ampulle und die Injektionsnadel auf. Diese gemeinsame Anschlussvorrichtung umfasst zum Beispiel eine Bohrung oder eine Hohlwelle, in die die Injektionsnadel und / oder die Ampulle zumindest teilweise einfügbar sind, oder einen Fortsatz, an den die Injektionsnadel und / oder die Ampulle anschließbar sind. Selbstverständlich kann die Anschlussvorrichtung auch anders ausgestaltet sein.

Die Kopplungseinheit, die mit dem Kupplungselement und der Anschlussvorrichtung versehen ist, sowie die Ampulle und die Injektionsnadel bilden im Betrieb des medizinischen Handgriffelements somit eine Einheit, auf die im Wesentlichen dasselbe Drehmoment einwirkt und die damit mit der im Wesentlichen selben Drehzahl rotieren. Anders ausgedrückt ist die Einheit zur Übertragung und / oder Aufnahme eines einheitlichen, gemeinsamen Drehmoments und einer einheitlichen, gemeinsamen Drehzahl ausgebildet.

In einem besonders bevorzugten Ausführungsbeispiel ist die Anschlussvorrichtung der Kopplungseinrichtung als Hohlwelle mit je einem Kupplungselement zur Drehmomentübertragung für die Ampulle und die Injektionsnadel ausgebildet. Dieser Aufbau gewährleistet eine besonders zuverlässige Drehmomentübertragung, eine stabile Lagerung und eine hohe Laufruhe der Injektionsnadel und der Ampulle.

Ist die Kopplungseinrichtung als Hohlwelle ausgebildet, so umgibt sie die Ampulle und die Injektionsnadel zumindest teilweise. Die Kopplungseinrichtung bzw. die Anschlussvorrichtung kann jedoch zumindest teilweise auch zwischen der Ampulle und der Injektionsnadel angeordnet sein, zum Beispiel wenn die Anschlussvorrichtung ein Gewinde aufweist, auf das die Injektionsnadel geschraubt wird.

Um die drehfeste Verbindung zwischen der Ampulle und der Injektionsnadel noch weiter zu unterstützen, ist in einem Ausführungsbeispiel vorgesehen, an der Kopplungsvorrichtung ein Spannelement vorzusehen, das die Verbindung dieser beiden Elemente verstärkt. Bevorzugt ist das Spannelement an einem Kupplungselement vorgesehen oder als Teil davon ausgebildet, so dass es in vorteilhafter Weise im unmittelbaren Bereich der Drehmomentübertragung angeordnet ist. Besonders bevorzugt weist das Spannelement zumindest eine konische Fläche an der Innenseite der als Anschlussvorrichtung dienenden Hohlwelle auf. Die konische Fläche ist derart ausgebildet, dass sie einen Bauteil einer in die Hohlwelle eingeführten Injektionsnadel, insbesondere einen Abschnitt der Nadelschürze, gegen die Ampulle drückt. Diese durch Teile der Injektionsnadel und das Spannelement in der Hohlwelle gebildete Spannvorrichtung stellt eine sehr einfache und effektive Verstärkung der drehfesten Verbindung zwischen der Ampulle und der Injektionsnadel dar.

Die Antriebsvorrichtung des medizinischen Handgriffelements zur Injektion einer medizinischen Substanz umfasst zumindest ein ein Drehmoment übertragendes, rotierendes Bauteil, wie eine Welle, ein oder mehrere Zahnräder, ein Getriebe etc. In einem Ausführungsbeispiel weist die Antriebsvorrichtung auch einen Motor, zum Beispiel einen Elektromotor oder einen mit einem Fluid, insbesondere mit Druckluft, betriebenen Motor auf. Der Motor ist entweder im medizinischen Handgriffelement angeordnet oder er ist als separates Bauteil ausgebildet und über eine Anschlussvorrichtung am medizinischen Handgriffelement mit diesem verbindbar. Besonders bevorzugt ist der Antriebsmotor als Teil einer dentalen Behandlungseinheit, zum Beispiel einer tragbaren Antriebs- und Steuereinheit oder eines Dentalstuhls, ausgebildet, so dass das medizinische Handgriffelement zur Injektion einer medizinischen Substanz über diese Behandlungseinheit antreibbar und gegebenenfalls steuerbar ist. Die Steuerung beinhaltet insbesondere die Auswahl und / oder Einstellung von Betriebsparametern durch den Anwender. Die Versorgung des Motors mit Strom erfolgt entweder über ein Stromversorgungsnetz oder mittels Batterie, die in einem bevorzugten Ausführungsbeispiel gemeinsam mit dem Motor im Handgriffelement angeordnet ist, so dass das Handgriffelement als kabelloses Gerät ohne Verbindung zu einer externen Einheit ausgebildet ist.

Die Fördervorrichtung des medizinischen Handgriffelements zur Injektion einer medizinischen Substanz ist in einem Ausführungsbeispiel als manuell bedienbare Fördervorrichtung ausgebildet, zum Beispiel als dreh- oder schwenkbarer Hebel, über den eine Zahnstange in Richtung der Ampulle bewegbar ist. Die Ampulle weist an einem Ende eine verschiebbare Verschlusskappe auf, die direkt oder indirekt durch die Zahnstange verschoben wird, wodurch der Inhalt der Ampulle aus dieser gedrückt wird. Alternativ ist eine motorisch antreibbare Fördervorrichtung vorgesehen, bei der ein Antriebsmotor eine Zahnstange oder einen Kolben in Richtung der Ampulle verschiebt.

Das medizinische Handgriffelement zur Injektion einer medizinischen Substanz ist in einem Ausführungsbeispiel als gerades, längliches Handgriffelement oder Handstück ausgebildet. Selbstverständlich kann das Handgriffelement jedoch auch andere Formen aufweisen, zum Beispiel eine gewinkelte, insbesondere eine pistolenförmige, Form oder es ist als Winkelstück mit einem gewinkelt angeordneten Vorder- oder Kopfabschnitt ausgebildet.

Ein weiterer Nachteil des Handgriffelements aus der US 4,787,893 besteht darin, dass zum Beladen des Handgriffelements mit der Ampulle und der Injektionsnadel das Vorderteil des Handgriffelements, das die Ampulle aufnimmt, vom Griffteil getrennt, die Ampulle in das Vorderteil eingesetzt, das Vorderteil wieder am Griffteil befestigt und anschließend von außen die Injektionsnadel an das Vorderteil des Handgriffelements angeschlossen und durch den Gummistopfen der Ampulle durchgesteckt werden muss. Dieser Beladevorgang ist für den Anwender umständlich und zeitaufwendig.

Dieser Nachteil wird dadurch gelöst, dass an einem Ende des Handgriffelements eine, bevorzugt kreisrunde, Aufnahmeöffnung vorgesehen ist, die derart ausgebildet ist, dass die Ampulle von diesem Ende des Handgriffelements durch die Aufnahmeöffnung in das Handgriffelement einfügbar ist. Es entfällt damit in vorteilhafter Weise beim Beladen des medizinischen Handgriffelements die Notwendigkeit, das Handgriffelement zerlegen zu müssen, und es wird generell der Beladevorgang des medizinischen Handgriffelements erleichtert.

In einem Ausführungsbeispiel befindet sich die Aufnahmevorrichtung oder Anschlussvorrichtung, in der die Ampulle nach dem Einschieben in das medizinische Handgriffelement angeordnet ist, vollstandig im medizinischen Handgriffelement und ist von einer Außenhülse des Handgriff elements umgeben. Dadurch wird in vorteilhafter Weise eine Schutzfunktion für den Anwender vor der rotierenden Ampulle erzielt.

In einem anderen Ausführungsbeispiel ist die Aufnahmeöffnung als Teil der Anschlussvorrichtung der Kopplungseinrichtung ausgebildet ist. Mit dem Einfügen der Ampulle durch die Aufnahmeöffnung wird damit die drehfeste Kopplung der Ampulle mit der Injektionsnadel zumindest eingeleitet.

Ein weiterer Nachteil des in der Patentschrift US 4,787,893 beschriebenen Injektionssystems besteht darin, dass es keine durchgehende Hygienekette und somit keinen vollständigen Schutz des Anwenders vor Verletzungen und Kontaminationen, zum Beispiel durch versehentliches Stechen mit der benutzten Nadel, bietet.

Dieser Nachteil wird durch ein Injektionssystem mit einer Ampulle, einer mit der Ampulle verbindbaren Injektionsnadel und einer Verriegelungsvorrichtung gelöst, wobei eine unlösbare Verriegelungsvorrichtung zur Verriegelung der Ampulle mit der Injektionsnadel vorgesehen ist. Unter einer unlösbaren Verriegelungsvorrichtung wird jede Verriegelungsvorrichtung verstanden, die so ausgebildet ist, dass eine einmalige Verriegelung oder Verbindung zwischen der Ampulle und der Injektionsnadel herstellbar ist, wobei die Verriegelung zerstörungsfrei nicht mehr lösbar ist. Die Verriegelungsvorrichtung ist somit nicht dafür bestimmt und nicht entsprechend ausgebildet, dass sie wieder trennbar ist. Die Verbindung der Ampulle mit der Injektionsnadel ist somit für den Anwender untrennbar.

Mit dieser Ausführung wird erreicht, dass nach dem Verbinden der Ampulle mit der Injektionsnadel und somit insbesondere nach der Injektion der medizinischen Substanz in das Zielgewebe und nach dem Kontakt des Injektionssystems mit dem Patienten jenes Ende der Kanüle der Injektionsnadel, das mit der Ampulle verbunden ist und das zum Beispiel durch das Septum in den Innenraum der Ampulle ragt, auch weiterhin in oder an der Ampulle verbleibt. Der Anwender wird damit vor Verletzungen durch dieses Kanülenende geschützt.

Ein entsprechendes Verfahren zum Entfernen einer Ampulle und einer Injektionsnadel aus einer Injektionsvorrichtung, zum Beispiel aus einem medizinischen Handgriffelement oder aus einem Spritzengestell, umfasst die Schritte:
- zur Verfügung stellen einer Injektionsvorrichtung mit einer Ampulle und einer Injektionsnadel, wobei die Ampulle und Injektionsnadel durch eine unlösbare Verriegelungsvorrichtung miteinander verbunden sind,
- gemeinsames Entfernen der Ampulle und der Injektionsnadel aus der Injektionsvorrichtung.

Bevorzugt erfolgt das Entfernen der untrennbar verbundenen Ampulle und Injektionsnadel durch eine Aufnahmeöffnung an einem Ende des Handgriffelements.

In einem Ausführungsbeispiel ist die Verriegelungsvorrichtung als formschlüssiger Mechanismus oder Rastmechanismus ausgebildet, wobei jeweils ein Teil der Verriegelungsvorrichtung an der Ampulle und an der Injektionsnadel vorgesehen ist. Der an der Ampulle vorgesehene Teil umfasst bevorzugt einen Rücksprung in der Außenhülse der Ampulle, insbesondere den Ampullenhals, der als eine Verengung in der Außenhülse ausgebildet ist. In diesen Rücksprung greift die Injektionsnadel bzw. ein speziell dafür vorgesehener Teil der Injektionsnadel formschlüssig oder rastend ein.

In einem Ausführungsbeispiel weist die Injektionsnadel eine Kanüle und eine Kanülenschürze auf, wobei die Kanülenschürze einen Halteabschnitt für die Kanüle und einen daran anschließenden Verbindungsabschnitt zur Verbindung der Injektionsnadel mit einer Injektionsvorrichtung, zum Beispiel einem medizinischen Handgriffelement oder einem Spritzengestell, und zur Verbindung mit der Ampulle aufweist. Bevorzugt ist zumindest ein Teil der unlösbaren Verriegelungsvorrichtung am Verbindungsabschnitt der Kanülenschürze vorgesehen ist. Aufgrund der geringen Abmessungen der Injektionsnadel und der Kanülenschürze ist die Ausformung der Kanülenschürze als Verbindungselement zur Injektionsvorrichtung und als Verriegelungsvorrichtung mit der Ampulle besonders vorteilhaft.

In einem Ausführungsbeispiel umfasst der an der Injektionsnadel vorgesehene Teil der Verriegelungsvorrichtung ein Formschluss- oder Rastelement zum Einrasten in den Rücksprung der Ampulle. Dieses auf dem Verbindungsabschnitt der Kanülenschürze angeordnete Rastelement ist bevorzugt als Rastnase mit einer Kontaktfläche ausgebildet, die über ihre gesamte Länge im Wesentlichen rechtwinkelig zum Verbindungsabschnitt angeordnet ist. Diese Ausbildung und eine ausreichende Länge der Kontaktfläche, so dass die Kontaktfläche und die Rastnase weit in den Rücksprung der Ampulle ragen, garantieren die Untrennbarkeit der Verriegelungsvorrichtung, in dem sie ein Lösen durch Herausziehen des Rastelements aus dem Rücksprung verhindern.

In einem bevorzugten Ausführungsbeispiel dient die Kanülenschürze bzw. dienen unterschiedliche Abschnitte, zum Beispiel Kupplungselemente, der Kanülenschürze zusätzlich auch als Verbindungselement mit einer Injektionsvorrichtung und / oder als Verdrehsicherung und / oder als Kupplungsvorrichtung zur Drehmomentübertragung und /oder als Spannvorrichtung zum Verspannen der Ampulle mit der Injektionsnadel. Der Vorteil dieser Ausführungsform liegt in der äußerst anwenderfreundlichen Ausgestaltung, da durch das Verbinden der Injektionsnadel mit der Ampulle bzw. durch das Einfügen der Injektionsnadel in die Injektionsvorrichtung mehrere Funktionen aktivierbar sind, ohne dass der Anwender dafür zusätzliche Maßnahmen ergreifen oder Handgriffe durchführen muss.

Selbstverständlich kann die unlösbare Verriegelungsvorrichtung zwischen der Ampulle und der Injektionsnadel auch anders ausgeführt sein und insbesondere einen anderen, unlösbaren Verriegelungsmechanismus als den oben beschriebenen formschlüssigen Mechanismus oder Rastmechanismus aufweisen. So ist in einem anderen Ausführungsbeispiel ein erstes Gewinde an einer Metallmanschette der Ampulle und ein zweites Gewinde an der Injektionsnadel vorgesehen, wobei zumindest eines der beiden Gewinde plastisch verformbar ist oder ein plastisch verformbares Bauteil aufweist oder mit einem Sperrelement versehen ist, so dass die Gewinde nach dem Verbinden nicht mehr voneinander lösbar sind.

Eine andere Lösung der Aufgabe, das Verletzungs- und Kontaminationsrisikos für den Anwender vor und nach dem Injektionsverfahren zu reduzieren, besteht darin, dass die Injektionsnadel eine Kanüle und eine Kanülenschürze aufweist, wobei die Kanüle ein erstes Ende zum Eindringen in das menschliche oder tierische Gewebe und ein zweites Ende zum Durchstoßen des Septums der Ampulle aufweist, die Kanülenschürze die Kanüle zumindest teilweise umgibt und die Kanülenschürze zum Schutz des Anwenders vor ungewollten Verletzungen über das zweite Ende der Kanüle ragt.

Durch diese Ausgestaltung wird der Anwender vor allem dann vor ungewünschten Verletzungen durch die Kanüle geschützt, wenn die Kanüle nicht mit einer Ampulle verbunden ist, also zum Beispiel beim Entnehmen der Injektionsnadel aus der Verpackung oder beim Verbinden der Injektionsnadel mit der Ampulle.

Bevorzugt ist der Innendurchmesser der Kanülenschürze zumindest in einem Abschnitt, insbesondere in jenem Abschnitt, in dem die Kanülenschürze über die Kanüle ragt, etwa so groß oder etwas größer als der Außendurchmesser der Ampulle, so dass die Ampulle in die Kanülenschürze einfügbar ist. Die Kanülenschürze dient damit in vorteilhafter Weise auch zur Aufnahme, Stützung und Führung der Ampulle.

Ein Ausführungsbeispiel eines Verfahren zum Be- und Entladen einer Injektionsvorrichtung, insbesondere eines medizinischen Handgriffelements zur Injektion einer medizinischen Substanz aus einer Ampulle in menschliches oder tierisches Gewebe, verringert ebenfalls die Verletzungsgefahr für den Anwender. Das Verfahren umfasst die Schritte:
- zur Verfügung stellen der medizinischen Injektionsvorrichtung,
- zur Verfügung stellen einer Ampulle mit einer medizinischen Substanz und einer Injektionsnadel,
- Verbinden der Ampulle mit der Injektionsnadel,
- Einfügen der Ampulle in die medizinische Injektionsvorrichtung.

Durch das Verbinden der Ampulle mit der Injektionsnadel vor dem Einfügen in die Injektionsvorrichtung wird der Verbindungsvorgang erleichtert. Üblicherweise wird zuerst die Ampulle in die Injektionsvorrichtung, insbesondere in ein medizinisches Handgriffelement, eingesetzt und dann die Injektionsnadel durch eine Aufnahmeöffnung in der Injektionsvorrichtung mit der Ampulle verbunden. Da die Aufnahmeöffnungen für die Injektionsnadeln oftmals sehr klein sind, fällt das Einführen der Injektionsnadel in diese Öffnung schwer, womit die Gefahr wächst, die Öffnung zu verfehlen und neben die Öffnung zu stechen. Dies wird durch das vorgeschlagene Verfahren verhindert.

In einem besonders bevorzugten Ausführungsbeispiel wird eine Injektionsnadel mit einer Kanülenschürze verwendet, die, wie weiter oben schon beschrieben, über das zweite Ende der Kanüle ragt. Damit ist die Gefahr, dass der Anwender sich während des Verbindens der Ampulle mit der Injektionsnadel verletzt, weiter verringert.

In einem anderen Ausführungsbeispiel wird die Ampulle durch eine Aufnahmeöffnung an einem Ende der Injektionsvorrichtung, insbesondere des Handgriffelements, eingefügt und / oder die Ampulle mit der Injektionsnadel durch eine Kopplungseinrichtung zur Vermeidung einer Relativbewegung gesichert.

In einem weiteren Ausführungsbeispiel werden die Ampulle und die mit ihr verbundene Injektionsnadel auch wieder gemeinsam von der medizinischen Injektionsvorrichtung getrennt. Damit wird erreicht, dass jenes Ende der Kanüle der Injektionsnadel, das mit der Ampulle verbunden ist, auch beim Trennen von der Injektionsvorrichtung in der Ampulle verbleibt. Der Anwender wird damit vor Verletzungen durch dieses Kanülenende geschützt.

Eine weitere Maßnahme zur Vermeidung unerwünschter Verletzungen des Anwenders durch die Injektionsnadel besteht darin, eine Injektionseinheit umfassend eine Injektionsnadel mit einer Kanüle und einer Kanülenschürze, ein Schutzelement, das die Injektionsnadel zumindest teilweise umgibt, und eine Verbindungsvorrichtung, insbesondere eine Rastvorrichtung, mit zwei Fixpositionen zu schaffen, wobei eine der beiden Fixpositionen als unlösbare Fixposition ausgebildet ist und wobei die Injektionsnadel und das Schutzelement zur Einnahme der beiden Fixpositionen verschiebbar zueinander ausgebildet sind.

Wird die Injektionsnadel in die unlösbare Fixposition geschoben, so sind das Schutzelement und die Injektionsnadel nicht mehr voneinander trennbar. Das Schutzelement umgibt bevorzugt jenes Ende der Kanüle der Injektionsnadel, das in das Gewebe des Patienten eindringt. Nach dem Abschluss des Injektionsvorgangs wird die Injektionsnadel, bevorzugt noch mit der Injektionsvorrichtung verbunden, in das Schutzelement geschoben, um die unlösbare Fixposition einzunehmen. Anschließend können die Injektionsnadel und das Schutzelement gemeinsam entsorgt werden bzw. gegebenenfalls vorher gemeinsam von der Injektionsvorrichtung gelöst werden. Durch die unlösbare Verbindung zwischen der Injektionsnadel und dem Schutzelement besteht weder beim Entsorgen noch beim Trennen der Injektionsvorrichtung die Gefahr, dass der Anwender versehentlich die Injektionsnadel vom Schutzelement löst und sich mit der gebrauchten Injektionsnadel verletzt.

In einem besonders bevorzugten Ausführungsbeispiel wird als Schutzelement zumindest ein Teil der Verpackung der Injektionsnadel, zum Beispiel eines Verpackungsbehälters, verwendet. Die lösbare Fix- oder Rastposition der Verbindungsvorrichtung ist dabei jene Position, in der sich die Injektionsnadel vor dem Gebrauch befindet. Der Anwender löst die Injektionsnadel vor der Anwendung aus der Verpackung / dem Schutzelement und verbindet sie mit der Ampulle und / oder der Injektionsvorrichtung. Nach dem Ende der Injektion schiebt er die Injektionsnadel wieder in die Verpackung / das Schutzelement, wobei er diesmal die unlösbare Fixposition auswählt. Dieses Ausführungsbeispiel hat den Vorteil, dass ein einziges Element sowohl als Verpackung als auch als Schutzelement dient.

In einem Ausführungsbeispiel werden die Fixpositionen durch Rücksprünge oder Nuten gebildet und umfasst die Verbindungsvorrichtung des Weiteren ein Eingriffselement, insbesondere ein Rastelement, zum Eingreifen in die Fixpositionen, wobei bevorzugt die Fixpositionen am Schutzelement und das Eingriffselement an der Injektionsnadel, insbesondere an der Kanülenschürze, vorgesehen sind. Diese Ausführungsform ermöglicht eine zuverlässige Fixierung in den Fixpositionen, bei gleichzeitig guter Verschiebbarkeit und einfacher Herstellung der Verbindungsvorrichtung.

In einem Ausführungsbeispiel wird die Unlösbarkeit einer der beiden Fixpositionen durch eine im Wesentlichen rechtwinkelig angeordnete Kante erzielt, die so ausgebildet ist, dass das Rastelement nicht darüber bewegbar ist, wohingegen die andere, lösbar ausgebildete Fixposition zumindest eine stetig ansteigende Seitenwand aufweist, so dass ein darin aufgenommenes Rastelement zum Beispiel herausziehbar ist.

In einem weiteren Ausführungsbeispiel weist das Schutzelement an seiner Außenseite Eingriffsmittel für einen Nadelwechsler auf, so dass das Entnehmen der Injektionsnadel aus dem Schutzelement und das Einfügen der Injektionsnadel in das Schutzelement mit Hilfe eines Nadelwechslers durchgeführt werden kann. Die Eingriffsmittel sind insbesondere als Rücksprung, Nut oder Ringnut ausgebildet.

Ein entsprechendes Verfahren zur Entsorgung einer Injektionsnadel umfasst die Schritte:
- zur Verfügung stellen einer Injektionsnadel,
- zur Verfügung stellen eines Schutzelements,
- Einfügen der Injektionsnadel in das Schutzelement und
- unlösbares Verbinden der Injektionsnadel mit dem Schutzelement.

In einem besonders bevorzugten Ausführungsbeispiel ist die unlösbar mit dem Schutzelement verbundene Injektionsnadel an ihrem anderen, nicht im Schutzelement aufgenommenen Kanülenende mit einer Ampulle verbunden, insbesondere durch eine unlösbare Verriegelungsvorrichtung, wie sie weiter oben schon beschrieben wurde, so dass damit beide Enden der Kanüle beim Entsorgen der Injektionsnadel nicht frei liegen. Dadurch wird die Verletzungsgefahr für den Anwender beim Trennen der Injektionsnadel von der Injektionsvorrichtung und beim Entsorgen weiter verringert.

Alternativ wird bei diesem Verfahren eine Injektionsnadel mit einer ebenfalls weiter oben bereits beschriebenen Kanülenschürze, die über das zweite, nicht im Schutzelement aufgenommene Kanülenende ragt, verwendet, wodurch die Verletzungsgefahr ebenfalls reduziert wird.

Die Erfindung wird nachfolgend anhand bevorzugter Ausführungsbeispiele und Bezug nehmend auf die beigefügten Zeichnungen erläutert:
Figur 1 zeigt eine Außenansicht eines Ausführungsbeispiels eines medizinischen Handgriffelements zur Injektion einer medizinischen Substanz.
Figur 2 zeigt eine Schnittdarstellung durch das medizinische Handgriffelement der Figur 1.
Figur 3 zeigt einen vergrößerten Ausschnitt des Kopfabschnitts des medizinischen Handgriffelements zur Injektion einer medizinischen Substanz der Figur 2.
Figur 4 zeigt eine Schnittdarstellung eines zweiten Ausführungsbeispiels eines Kopfabschnitts eines medizinischen Handgriffelements zur Injektion einer medizinischen Substanz.
Figur 5 zeigt eine Schnittdarstellung eines dritten Ausführungsbeispiels eines Kopfabschnitts eines medizinischen Handgriffelements zur Injektion einer medizinischen Substanz.
Figur 6 zeigt eine Schnittdarstellung eines vierten Ausführungsbeispiels eines Kopfabschnitts eines medizinischen Handgriffelements zur Injektion einer medizinischen Substanz.
Figur 7 zeigt eine perspektivische Ansicht eines Ausführungsbeispiels einer Kanülenschürze einer Injektionsnadel.
Figur 8 zeigt ein Ausführungsbeispiel einer Injektionseinheit und einer davon getrennten Ampulle.
Figur 9 zeigt die Injektionseinheit und die Ampulle aus Figur 8 miteinander verbunden.
Figur 10 zeigt ein Ausführungsbeispiel eines Nadelwechslers mit einer eingespannten Injektionseinheit.
Figur 11 zeigt eine vergrößerte Detailansicht eines Ausführungsbeispiels einer Verbindungsvorrichtung in einer Injektionseinheit zur Verbindung einer Injektionsnadel mit einem Schutzelement.
Figur 12 zeigt ein Ausführungsbeispiel eines Nadelhalters und einer damit verbundenen Injektionseinheit sowie ein medizinisches Handgriffelement, in das die Injektionseinheit einfügbar ist.

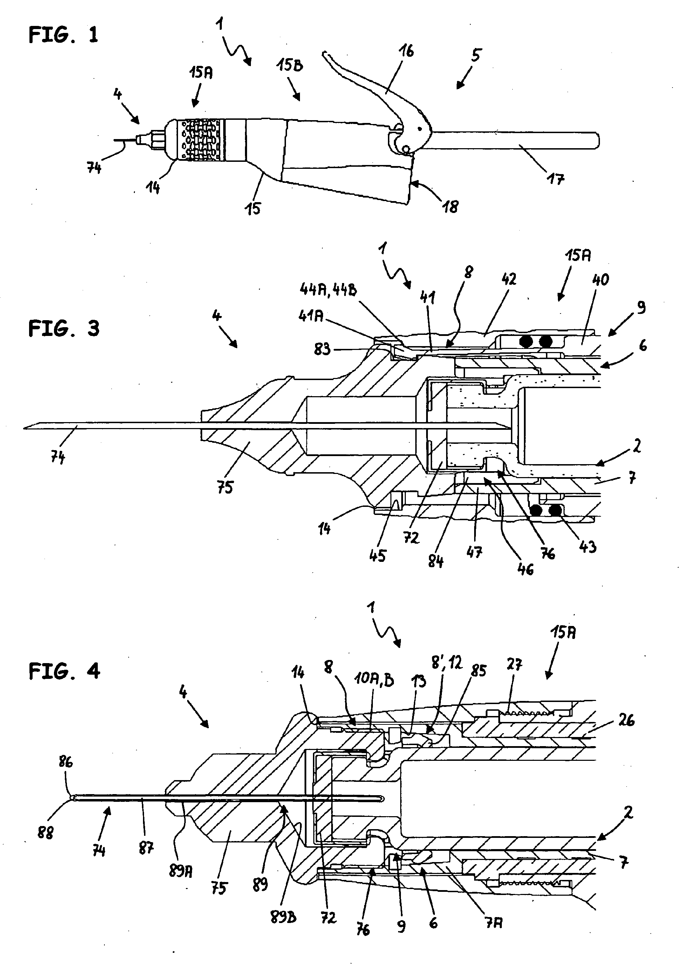

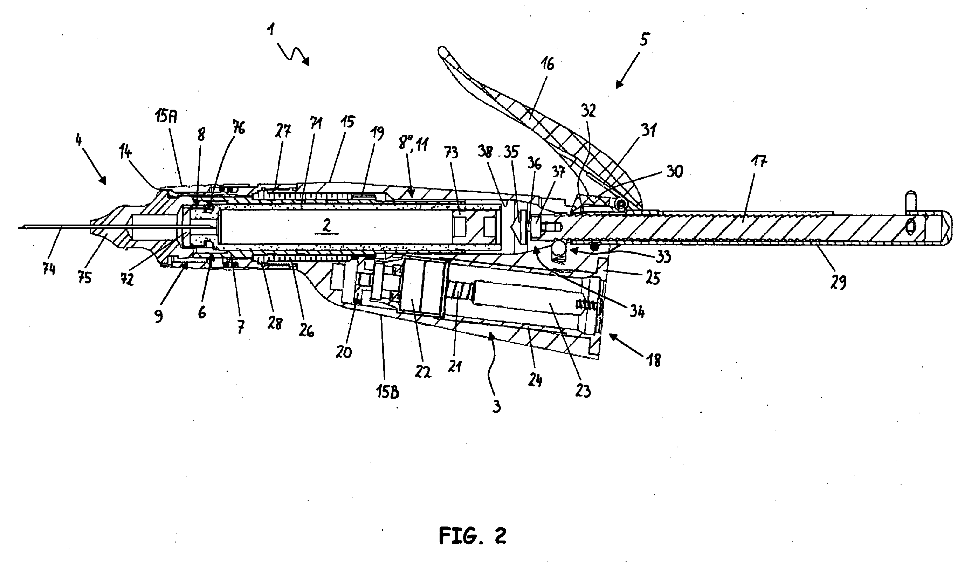

Das in Figur 1 dargerstellte Ausführungsbeispiel eines medizinischen Handgriffelements 1 dient als dentales, intraossäres Handstück insbesondere zur Herstellung einer Bohrung in einem Kieferknochen und zur anschließenden Injektion einer medizinischen Substanz, insbesondere eines Anästhetikums, in die Bohrung und den Knochen. Das längliche, rohrförmige Handgriffelement 1 weist eine Außenhülse 15 mit einem Griffabschnitt 15B und einem daran anschließenden Kopfabschnitt 15A auf. Der Griffabschnitt 15B und der Kopfabschnitt 15A sind über ein Gewinde 27 (siehe Figur 2) miteinander verbunden. Am Ende des Kopfabschnitts 15A des Handgriffelements 1 ist eine, bevorzugt kreisrunde, Aufnahmeöffnung 14 vorgesehen, durch die eine Ampulle 2 mit dem Anästhetikum in das Handgriffelement 1 einführbar ist. An die Ampulle 2 ist eine Injektionsnadel 4 angeschlossen, die aus dem Handgriffelement 1 herausragt.

Die Injektionsnadel 4 umfasst eine längliche Kanüle 74, die in bekannter Weise sowohl als Bohrinstrument zur Ausformung der Bohrung in den Knochen als auch zur Abgabe des Anästhetikums dient. Sie weist dazu an einem Endabschnitt zumindest eine Schneidkante 86 (siehe Figur 4) auf, die, wenn die Injektionsnadel 4 in Rotation versetzt ist, Knochenspäne aus dem Knochen schneidet und so die Bohrung in den Knochen erzeugt. Die Kanüle 74 ist des Weiteren mit einem durch ihr Inneres verlaufenden Hohlraum 87, der als Kanal zur Leitung des Anästhetikums dient, und einer mit dem Hohlraum 87 verbundenen Öffnung 88 zur Abgabe des Anästhetikums an den Knochen ausgestattet. Die Kanüle 74 ist des Weiteren so geformt, dass eines ihrer Enden durch den Gummistopfen oder das Septum 72 der Ampulle 2 in den Innenraum der Ampulle 2 mit dem Anästhetikum eindringen kann.

Zum Austreiben des Anästhetikums oder der medizinischen Substanz aus der Ampulle 2 ist eine Fördervorrichtung 5 vorgesehen, die gemäß dem Ausführungsbeispiel nach den Figuren 1 und 2 als manuell betätigbare Fördervorrichtung ausgebildet ist. Die Fördervorrichtung 5 umfasst einen dreh- oder schwenkbaren Hebel 16, der über eine Mitnehmervorrichtung mit einem Kolben oder einer Zahnstange 17 verbunden ist, die in Richtung der Ampulle 2 verschiebbar im Handgriffelement 1 aufgenommen sind, so dass durch das Verschieben der Zahnstange 17 das Anästhetikum aus der Ampulle 2 und durch die Kanüle 74 gefördert wird.

An jenem Ende des Handgriffelements 1, das der Öffnung 14 gegenüber liegt, ist eine Anschlussvorrichtung 18 vorgesehen, die zum Anschluss des Handgriffelements 1 an einen Antriebsmotor dient. Der Antriebsmotor versetzt über die im Inneren des Handgriffelements 1 angeordnete Antriebsvorrichtung 3 die Injektionsnadel 4 und die Ampulle 2 in Rotation. Die Anschlussvorrichtung 18 ist zum Beispiel als Steck-, Formschluss- oder Schraubverbindung ausgebildet.

Die Figur 2 zeigt eine vergrößerte Schnittdarstellung des medizinischen Handgriffelements 1 der Figur 1 mit einer vollständig im Inneren des Handgriffelements 1 aufgenommenen Ampulle 2 und einer mit der Ampulle 2 verbundenen und zumindest teilweise in dem Handgriffelement 1 aufgenommenen Injektionsnadel 4.

Die Ampulle 2 ist in eine Anschlussvorrichtung 6, die eine Hohlwelle 7 umfasst, eingeschoben. Die Anschlussvorrichtung 6 befindet sich in einer Innenbohrung 38 des Handgriffelements 1. Die Innenbohrung 38 weist zwei Öffnungen an ihren entgegen gesetzten Enden für die Fördervorrichtung 5 und für das Einfügen der Ampulle 2 und der Injektionsnadel 4 auf. Die Öffnung der Innenbohrung 38 zum Einfügen der Ampulle 2 und der Injektionsnadel 4 ist mit der Aufnahmeöffnung 14 des Handgriffelements 1 verbunden oder ist Teil der Aufnahmeöffnung 14.

Der Innendurchmesser der Hohlwelle 7 entspricht in etwa dem Außendurchmesser der Außenhülle 71 der Ampulle 2, so dass die Ampulle 2 fest sitzend in der Hohlwelle 7 aufgenommen ist. Die Länge der Hohlwelle 7 ist etwas geringer als die Länge der Ampulle 2, so dass die Ampulle 2 an ihren beiden Enden aus der Hohlwelle 7 herausragt.

An der Außenseite der Hohlwelle 7 ist ein Zahnrad 19 vorgesehen, das Teil der Antriebsvorrichtung 3 zum rotierenden Antrieb der Ampulle 2 und der Injektionsnadel 4 ist. Das Zahnrad 19 kämmt mit einem weiteren Zahnrad 20, das auf eine Wellenanordnung 21 aufgepresst ist. Die Wellenanordnung 21 umfasst ein Planetengetriebe 22 und ist mit einem Mitnehmer 23 verbunden. Zumindest ein Teil der Wellenanordnung 21 und der Mitnehmer 23 sind in einer Lagerhülse 24 aufgenommen, deren flanschartiges Ende 25 Teil der Anschlussvorrichtung 18 ist. An die Anschlussvorrichtung 18 ist ein Antriebsmotor, zum Beispiel ein bürstenloser Elektromotor, anschließbar, dessen vom Planetengetriebe 22 untersetzte Drehbewegung auf die Hohlwelle 7 übertragen wird. Die rotierbare Hohlwelle 7 ist in einer Lagerhülse 26 aufgenommen und darin gleitend gelagert. Die Lagerhülse 26 ist über Fortsätze 28 an einem drehfesten Bauteil des Handgriffelements 1, zum Beispiel an der Außenhülse 15, fixiert.

Außer dem Zahnrad 19 ist die gesamte Antriebsvorrichtung 3 in einer Verbreiterung der Außenhülse 15, an der Unterseite des Handgriffelements 1 angeordnet. Die Verbreiterung der Außenhülse 15 nimmt in Richtung des Kopfabschnitts 15A ab, wodurch die Antriebsvorrichtung 3 schräg, in einem spitzen Winkel zur Längsachse des Handgriffelements 1 angeordnet ist.

Die Fördervorrichtung 5 umfasst einen manuell bedienbaren, schwenkbaren Hebel 16 sowie eine Zahnstange 17, die in einer Schutzhülse 29 aufgenommen ist. Am Hebel 16 und mit diesem verbunden ist eine Klinke 30 vorgesehen, deren Vorderende keilförmig ausgebildet ist. Die Klinke 30 ist durch eine Feder 31 in Richtung der Zahnstange 17 vorgespannt. Durch Schwenken des Hebels 16 abwechselnd in Richtung der Öffnung 14 und in die Gegenrichtung wird die Zahnstange 17 in Richtung der Ampulle 2 verschoben. Dies geschieht, indem das keilförmige Vorderende der Klinke 30 durch eine Öffnung 32 der Schutzhülse 29 zwischen zwei Zähne der Zahnstange 17 eingreift und diese verschiebt. Wird der Hebel 16 in die Gegenrichtung geschwenkt, so löst sich das keilförmige Vorderende der Klinke 30 von der Zahnstange 17, um bei der nächsten Bewegung des Hebels 16 in Richtung der Öffnung 14 wieder in die Zahnstange 17 einzugreifen.

Eine Fixiervorrichtung 33, die zum Beispiel eine Kugel und eine die Kugel vorspannende Feder umfasst, verhindert während der Betätigung des Hebels 16 und somit während des Fördervorgangs des Anästhetikums das Zurückrutschen der Zahnstange 17 in Richtung der Anschlussvorrichtung 18, in dem die Kugel der Fixiervorrichtung 33 zwischen zwei Zähne der Zahnstange 17 einrastet.

Das der Öffnung 14 zugewandte Ende der Zahnstange 17 ist als drehbares Schubteil 34 mit einem Keil 35 ausgebildet. Der Keil 35 ist auf einer Welle 36 befestigt, die drehbar mit einem Lager 37 verbunden ist. Das Lager 37 ist in einer Aufnahme der Zahnstange 17 fixiert. Wird die Zahnstange 17 in Richtung der Öffnung 14 bewegt, so greift der Keil 35 an der verschiebbaren Verschlusskappe 73 der Ampulle 2 an und schiebt diese in Richtung des Septums 72, wodurch die in der Ampulle 2 enthaltene Substanz aus der Ampulle 2 in die Kanüle 74 gepresst wird. Da die Ampulle 2 während des Betriebs des Handgriffelements 1 rotiert, ist es notwendig, den Keil 35 rotierbar zu lagern, um zu verhindern, dass die Ampulle 2 durch die Zahnstange 17 gebremst wird und es dadurch zu einer unerwünschten Relativbewegung zwischen der Ampulle 2 und der Injektionsnadel 4 kommt.

Die Aufnahmeöffnung 14 ist so bemessen, dass die Injektionsnadel 4 mit der Kanülenschürze 75, die die Kanüle 74 zumindest teilweise umgibt, durch die Aufnahmeöffnung 14 in das Handgriffelement 1 einfügbar ist. Die Injektionsnadel 4 ist über eine Kupplungsvorrichtung 8 mit dem Handgriffelement 1 verbunden, wobei Teile der Kupplungsvorrichtung 8 an der Injektionsnadel und weitere Kupplungselemente am Handgriffelement 1 vorgesehen sind. Die Kupplungsvorrichtung 8 dient jedoch nicht nur dem Anschluss der Injektionsnadel 4 an das Handgriffelement 1, sondern auch der Drehmomentübertragung von der Anschlussvorrichtung 6 auf die Injektionsnadel 4.

Eine weitere Kupplungsvorrichtung 8" ist an der als Hohlwelle 7 ausgebildeten Anschlussvorrichtung 6 vorgesehen. Die Kupplungsvorrichtung 8" umfasst mehrere Federlaschen 11, die radial nach Innen, in Richtung der Ampulle 2 vorgespannt sind und die Ampulle 2 in und an der Anschlussvorrichtung 6 befestigen. Auch diese Kupplungsvorrichtung 8" dient zusätzlich zur Übertragung des Drehmoments von der Anschlussvorrichtung 6 auf die Ampulle 2.

Aus der Figur 2 ist erkennbar, dass die Kopplungseinrichtung 9 zur drehfesten Verbindung der Ampulle 2 mit der Injektionsnadel 4 eine Relativbewegung zwischen diesen beiden Elementen unterbindet, in dem sie sicherstellt, dass durch die Kupplungsvorrichtungen 8 und 8" das von der Antriebsvorrichtung 3 erzeugte Drehmoment zuverlässig sowohl auf die Ampulle 2 als auch auf die Injektionsnadel 4 übertragen wird und dass die Ampulle 2 und die Injektionsnadel 4 über die Anschlussvorrichtung 6 mit der Hohlwelle 7 fest miteinander verbunden sind. Durch und gemeinsam mit der Kopplungseinrichtung 9 bilden die Injektionsnadel 4 und die Ampulle 2 somit eine Einheit zur Übertragung und / oder Aufnahme eines einheitlichen, gemeinsamen Drehmoments und einer einheitlichen, gemeinsamen Drehzahl.

Die Figuren 3 - 6 zeigen verschieden Ausführungsbeispiele des Kopfabschnitts medizinischer Handgriffelemente zur Injektion einer medizinischen Substanz aus einer Ampulle 2, wobei Figur 3 eine vergrößerte Darstellung des Kopfabschnitts 15A des Handgriffelements 1 der Figur 2 ist.

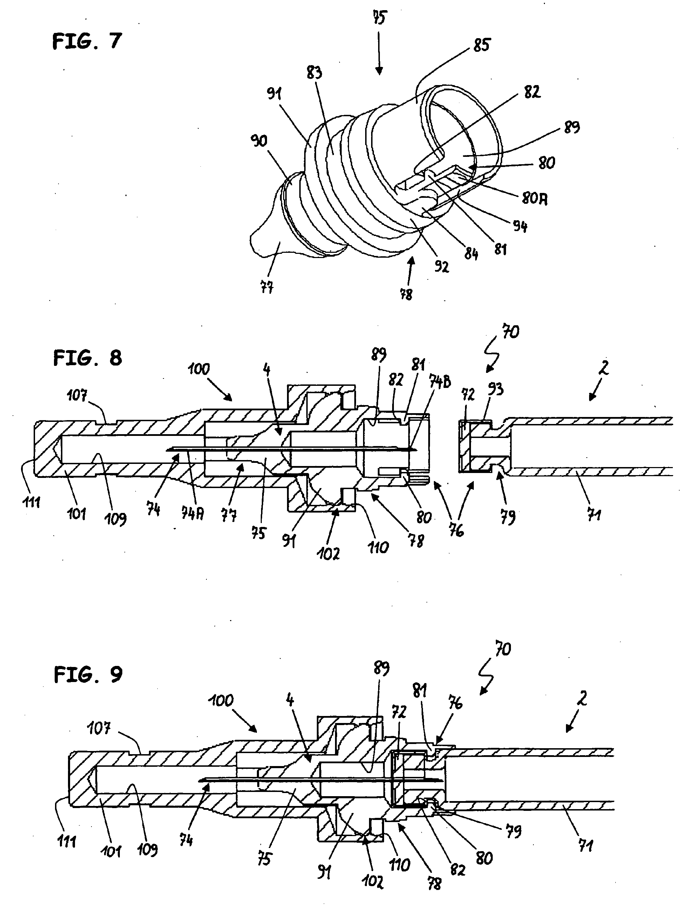

Die Kupplungsvorrichtung 8 zur Befestigung der Injektionsnadel 4 am Handgriffelement 1 und zur Drehmomentübertragung auf die Injektionsnadel 4 umfasst eine Spannhülse 40 mit mehreren Federarmen 41, an deren Ende Rastelemente 41 A, zum Beispiel in Form von Rastnasen, vorgesehen sind. Als Gegenkupplungselement verläuft rund um die Kanülenschürze 75 der Injektionsnadel 4 eine Ringnut 83 (siehe auch Figur 7), in die die Rastelemente 41A eingreifen, so dass die Injektionsnadel 4 axial am Handgriffelement 1 fixiert ist. Die Federarme 41 sind von einem Stellelement, zum Beispiel einer Schiebehülse 42, umgeben, die entlang der Längsachse des Handgriffelements 1 verschiebbar ist und durch ein Federelement 43 in Richtung der Aufnahmeöffnung 14 vorgespannt wird. An der Schiebehülse 42 sowie an den Federarmen 41 sind Kontaktflächen 44A, 44B vorgesehen, die derart geformt sind, dass die Rastelemente 41A durch die Schiebehülse 42 in die Ringnut 83 gedrückt wird.

Wird die Schiebehülse 42 gegen die Federkraft der Feder 43 in Richtung der Fördervorrichtung 5 verschoben, so werden die Kontaktflächen 44A, 44B voneinander getrennt und die Rastelemente 41 A können in einen Rücksprung 45 der Schiebehülse 42 radial nach außen ausweichen, wodurch sie aus der Ringnut 83 austreten und die Injektionsnadel 4 vom Handgriffelement 1 lösbar ist.

Die bis hierher beschriebenen Bauteile 41, 42, 43, 83 usw. der Kupplungsvorrichtung 8 bilden somit einen Kupplungsteil, der ausschließlich der Fixierung der Injektionsnadel 4 am Handgriffelement 1 dient. Die Übertragung des Drehmoments auf die Injektionsnadel 4 erfolgt gemäß diesem Ausführungsbeispiel über einen zweiten, davon getrennten Kupplungsteil mit anderen Elementen: Es sind dazu an der Hohlwelle 7 mehrere, bevorzugt vier, Mitnehmerelemente 46, zum Beispiel in Form von Aufnahmen oder Rücksprüngen, vorgesehen, die durch Trennelemente 47, zum Beispiel vier Stege, voneinander getrennt sind. Ist die Injektionsnadel 4 mit dem Handgriffelement 1 verbunden, so greifen ein oder mehrere Kupplungselemente 84 der Injektionsnadel 4 (siehe auch Figur 7), zum Beispiel Kupplungsnasen, die Teil der Kanülenschürze 75 sind, in die Mitnehmerelemente 46 der Hohlwelle 7 ein. Wird die Hohlwelle 7 in Rotation versetzt, so übertragen die Mitnehmerelemente 46 der Hohlwelle 7 die Rotationsbewegung und das Drehmoment auf die Kupplungselemente 84 und die Injektionsnadel 4.

Im Gegensatz dazu ist die in der Figur 4 dargestellte Ausführungsform der Kupplungsvorrichtung 8 so ausgebildet, dass die Fixierung der Injektionsnadel 4 am Handgriffelement 1 und die Drehmomentübertragung auf die Injektionsnadel 4 am selben Ort und durch die selben Bauteile erfolgt: Es weist dazu die Hohlwelle 7 an einem Ende einen Abschnitt 7A mit einem verbreitertem Durchmesser auf. An diesem Abschnitt 7A ist ein Innengewinde 10A vorgesehen, das mit einem Außengewinde 10B der Injektionsnadel 4, bevorzugt der Kanülenschürze 75, verschraubbar ist. Wird die Hohlwelle 7 in Rotation versetzt, so wird die Rotationsbewegung und das Drehmoment über das Gewinde 10A, 10B auf die Injektionsnadel übertragen. Selbstverständlich können anstelle der Gewindeverbindung auch andere bekannte Verbindungen vorgesehen sein, zum Beispiel Bajonettverbindungen, formschlüssige Verbindungen mit Spannlaschen etc.

Die Kopplungseinrichtung 9 der Figur 4 weist des Weiteren eine Spannvorrichtung 12 zum Verspannen der Ampulle 2 mit der Injektionsnadel 4 auf. Diese Spannvorrichtung 12 kann als zusätzliche Sicherungsvorrichtung zur drehfesten Verbindung zwischen der Injektionsnadel 4 und der Ampulle 2 dienen, sie kann alternativ jedoch auch anstelle der Kupplungsvorrichtung 8" (siehe Figur 2) vorgesehen sein. In diesem Fall erfolgt die Drehmomentübertragung von der Antriebsvorrichtung 3 und der Anschlussvorrichtung 6 auf die Ampulle 2 ebenfalls über die Spannvorrichtung 12, so dass die Spannvorrichtung 12 gleichzeitig auch als Kupplungsvorrichtung 8' dient.

Die Spannvorrichtung 12 weist ein Spannelement 13, zum Beispiel eine konische Fläche, an der Kopplungseinrichtung 9, insbesondere am verbreiterten Abschnitt 7A der Hohlwelle 7, auf. An der Injektionsnadel 4, insbesondere an der Kanülenschürze 75, ist ein Gegenspannelement 85, zum Beispiel in Form eines oder mehrerer Flügel, vorgesehen. Ist die Injektionsnadel 4, wie in Figur 4 dargestellt, in das Handgriffelement 1 eingefügt, dann kontaktiert und drückt die konische Fläche 13 den Flügel 85 radial nach Innen in Richtung der Ampulle 2. Der Flügel 85 und die Kanülenschürze 75 werden somit gegen die Ampulle 2 gepresst, wodurch eine Reibverbindung zwischen der Ampulle 2 und der Injektionsnadel 4 hergestellt wird. Diese Reibverbindung dient zur Verdrehsicherung und gegebenenfalls zur Übertragung des Drehmoments und der Rotationsbewegung auf die Ampulle 2.

In den Figuren 2 - 4 ist auch eine unlösbare Verriegelungsvorrichtung 76 zur untrennbaren Verriegelung der Ampulle 2 mit der Injektionsnadel 4 zu erkennen, die weiter unten, insbesondere in Zusammenhang mit den Figuren 7 - 9 beschrieben wird.

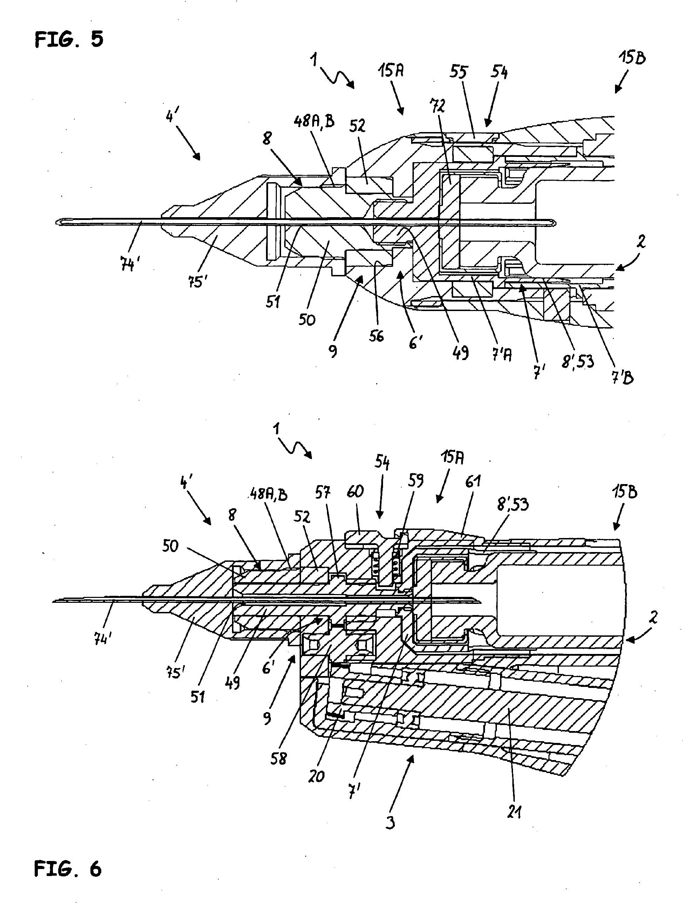

Die Figuren 5 und 6 zeigen den Kopfabschnitt 15A eines Handgriffelements 15 für einen anderen Injektionsnadeltypus, der mit dem Bezugszeichen 4' versehen ist. Diese Injektionsnadel 4' ist weit verbreitet und wird daher auch als Standardnadel bezeichnet. Sie umfasst eine Kanüle 74', deren Aufbau gleich der Kanüle 74 ist, und eine Kanülenschürze 75'. Die Kanülenschürze 75' ist mit einem Innengewinde 48A versehen, das zur Verbindung mit einem Gegengewinde 48B an einer Injektionsvorrichtung, zum Beispiel eine medizinischen Handgriffelement 1, vorgesehen ist.

Aufgrund des Aufbaus der Injektionsnadel 4' und der Kanülenschürze 75' ist es notwendig, die Kopplungseinrichtung 9 zur drehfesten Verbindung einer Ampulle 2 mit der Injektionsnadel 4' anders auszuführen, als dies für die Figuren 2 - 4 beschrieben wurde: Die Kopplungseinrichtung 9 gemäß Figur 5 umfasst wiederum eine Anschlussvorrichtung 6' mit einer Hohlwelle 7'. Die Hohlwelle 7' ist wie die Hohlwelle 7 aus der Figur 2 mit einem Zahnrad 19 verbunden, um durch die Antriebsvorrichtung 3 in Rotation versetzt zu werden, und sie ist mit einem ersten Kupplungselement 8" versehen, das die Ampulle 2 in der Hohlwelle 7' fixiert und die Drehmomentübertragung auf die Ampulle 2 sicherstellt. Unterschiedlich zur Hohlwelle 7 ist die Hohlwelle 7' jedoch zweigeteilt und weist zwei trennbare Abschnitte 7'A, 7'B auf. Am Abschnitt 7'A ist eine Kupplungsvorrichtung 8' mit mehrere Spannlaschen 53 vorgesehen, die nach innen, in Richtung der Längsachse des Handgriffelements 1 vorgespannt sind, und die die Ampulle 2 im Hohlwellenabschnitt 7'A fixieren.

Das der Injektionsnadel 4' zugewandte Ende der Hohlwelle 7' weist einen Fortsatz 49 auf, der mit einem Anschlusselement 50, das ebenfalls Teil der Kopplungseinrichtung 9 und der Anschlussvorrichtung 6' ist, zum Beispiel über ein Gewinde verbunden ist. An diesem Anschlusselement 50 ist ein Teil der Kupplungsvorrichtung 8 mit dem Außengewinde 48B zur Verbindung mit der Injektionsnadel 4' vorgesehen. Das Anschlusselement 50, der Fortsatz 49 als auch der an den Fortsatz 49 anschließende Wandabschnitt der Hohlwelle 7' weisen eine schmale Bohrung 51 auf, durch die die Kanüle 74' der Injektionsnadel 4' geführt ist, so dass die Kanüle 74' durch das Septum 72 der Ampulle 2 bis in den mit der zu injizierenden Substanz gefüllten Innenraum der Ampulle 2 gelangen kann. Das Anschlusselement 50 und der Fortsatz 49 sind durch ein Lager 52 drehbar im Handgriffelement 1 gelagert. Das Lager 52 ist in einem Lagersitz 56 des Kopfabschnitts 15A aufgenommen.

Die Übertragung des Drehmoments und der Rotationsbewegung auf die Injektionsnadel 4' und die Ampulle 2 erfolgt bei diesem Ausführungsbeispiel über die die Hohlwelle 7' mit der Kupplungsvorrichtung 8" (siehe Figur 2) und der Kupplungsvorrichtung 8', den Fortsatz 49, das Anschlusselement 50 und die Kupplungsvorrichtung 8 mit dem Gewinde 48A, 48B. Die durch die Kopplungseinrichtung 9 bewirkte drehfeste Verbindung zwischen der Ampulle 2 und der Injektionsnadel 4' wird durch die drehfeste Aufnahme der Ampulle 2 in der Hohlwelle 7' durch die Kupplungsvorrichtung 8" und die Kupplungsvorrichtung 8', die drehfeste Verbindung der Injektionsnadel 4' mit dem Anschlusselement 50 (die bei den Gewinden 48A, 48B zumindest in Drehrichtung der Gewinde 48A, 48B gegeben ist, wenn die Gewinde 48A, 48B bis zum Anschlag miteinander verschraubt sind) und die drehfeste Verbindung des Anschlusselements 50 mit dem Fortsatz 49 der Hohlwelle 7' gewährleistet.

Aufgrund der geschlossenen Ausformung der Hohlwelle 7' an dem der Injektionsnadel 4' zugewandten Ende mit dem davor angeordneten Fortsatz 49 ist es bei der Ausführungsform gemäß den Figur 5 nicht möglich, die Ampulle 2 vom Vorderende des Handgriffelements 1 in die Hohlwelle 7' zu schieben. Stattdessen ist das Handgriffelement 1 zweiteilig ausgebildet, so dass der Kopfabschnitt 15A vom Griffabschnitt 15B des Handgriffelements 1 trennbar ist, wobei im getrennten Zustand der Hohlwellenabschnitt 7'A im Kopfabschnitt 15A und der Hohlwellenabschnitt 7'B im Griffabschnitt 15B verbleibt. Nach der Trennung der beiden Teile 15A, 15B des Handgriffelements 1 kann die Ampulle 2 in den Hohlwellenabschnitt 7'A eingefügt werden und anschließend können die beiden der Kopfund der Griffabschnitt 15A, 15B wieder miteinander verbunden werden, wobei der über den Kopfabschnitt 15A und den Hohlwellenabschnitt 7'A hinausragende Teil der Ampulle 2 in den Hohlwellenabschnitt 7'B eingefügt wird. Anschließend kann die Injektionsnadel 4' auf das Anschlusselement 50 aufgeschraubt werden.

Die Verbindung der beiden Hohlwellenabschnitt 7'A, 7'B erfolgt durch teilweises Ineinander- bzw. Übereinanderschieben. Die Verbindung des Kopf- und des Griffabschnitts 15A, 15B wird durch eine Kupplungsvorrichtung 54 bewerkstelligt. Die Kupplungsvorrichtung 54 kann zum Beispiel nach außen vorgespannte Spannlaschen am Kopfabschnitt 15A aufweisen, die in Ausnehmungen des Griffabschnitts 15B eingreifen. Über einen elastischen federnden Lösemechanismus 55 werden die Spannlaschen aus dem Eingriff mit den Ausnehmungen gebracht, so dass Kopf- und der Griffabschnitts 15A, 15B voneinander getrennt werden können. Selbstverständlich können auch andere Kupplungsmechanismen zur Anwendung kommen.

In einem anderen, nicht dargestellten Ausführungsbeispiel ist die Hohlwelle nicht teilbar ausgebildet sondern weist eine Aufnahmeöffnung für die Ampulle am von der Injektionsnadel abgewandten Ende auf. Durch eine Teilung des Handgriffelements wie oben beschrieben in einen Kopf- und der Griffabschnitt 15A, 15B und dem Verbleib der gesamten Hohlwelle im Kopfabschnitt 15A wird diese Aufnahmeöffnung zugänglich und die Ampulle kann vom rückwärtigen Ende der Hohlwelle in diese eingeschoben werden.

Das Handgriffelement 1 und die Kopplungseinrichtung 9 gemäß der Figur 6 sind ebenfalls für den Anschluss einer Standardnadel 4' ausgelegt. Es ist wiederum ein Anschlusselement 50 mit einer Kupplungsvorrichtung 8 mit einem Gewinde 48A, 48B für den Anschluss der Injektionsnadel 4' vorgesehen. Das Anschlusselement 50 ist mit einem Fortsatz 49 der Hohlwelle 7' verbunden, wobei der Fortsatz 49 das Anschlusselement 50 vollständig durchsetzt. Ein Lager 52, zum Beispiel ein Gleitlager, lagert das Anschlusselement 50 und den Fortsatz 49. Eine Bohrung 51 für die Kanüle 74' der Injektionsnadel 4' durchsetzt den Fortsatz 49 und die anschließende Wand der Hohlwelle 7'.

Die Hohlwelle 7' als Teil der Anschlussvorrichtung 6' ist als kurze Aufnahme ausgebildet, in die nur einen Teil der Ampulle 2 einführbar ist. Mehrere Spannlaschen 53 der Kupplungsvorrichtung 8' fixieren die Ampulle 2 an der Hohlwelle 7'.

Die Übertragung der Drehbewegung und des Drehmoments von der Antriebsvorrichtung 3 auf die Injektionsnadel 4' und die Ampulle 2 erfolgt gemäß diesem Ausführungsbeispiel über den Fortsatz 49, der mit einem Zahnrad 57 versehen ist, einerseits auf die Hohlwelle 7', die Kupplungsvorrichtung 8' und die Ampulle 2 und andererseits auf das Anschlusselement 50, die Kupplungsvorrichtung 8" und die Injektionsnadel 4'. Ein Zwischenzahnrad 58 verbindet das auf der Welle 21 aufgepresste Zahnrad 20 mit dem Zahnrad 57. Das Zwischenzahnrad 58 kann zur Untersetzung der Drehzahl ausgebildet sein.

Die durch die Kopplungseinrichtung 9 bewirkte drehfeste Verbindung zwischen der Ampulle 2 und der Injektionsnadel 4' wird durch die drehfeste Aufnahme der Ampulle 2 in der Hohlwelle 7' durch die Kupplungsvorrichtung 8', die drehfeste Verbindung der Injektionsnadel 4' mit dem Anschlusselement 50 (die bei den Gewinden 48A, 48B zumindest in Drehrichtung der Gewinde 48A, 48B gegeben ist, wenn die Gewinde 48A, 48B bis zum Anschlag miteinander verschraubt sind) und die drehfeste Verbindung des Anschlusselements 50 mit dem Fortsatz 49 gewährleistet.

Zum Einsetzen der Ampulle 2 in die Hohlwelle 7' ist wiederum die Trennung des Handgriffelements 1 in seine beiden Abschnitte 15A, 15B notwendig. Die Hohlwelle 7' verbleibt nach der Trennung im Kopfabschnitt 15A, so dass die Ampulle 2 in Richtung des Fortsatzes 49 in die Hohlwelle 7' einschiebbar ist. Die Verriegelung des Kopfabschnitts 15A mit dem Griffabschnitt 15B erfolgt über eine Kupplungsvorrichtung 54, die ein durch eine Feder 59 vorgespanntes Formschlusselement 60 am Kopfabschnitt 15A und eine hülsenförmiges, den Griffabschnitt 15B zumindest teilweise umgebendes Gegenelement 61 mit einer Aufnahme zum Eingriff eines Teils des Formschlusselements 60 aufweist. Drückt ein Anwender auf das Formschlusselement 60 und verschiebt es gegen die Federkraft der Feder 59, so wird das Formschlusselement 60 aus der Aufnahme des Gegenelements 61 gelöst und die beiden Abschnitte 15A, 15B können voneinander getrennt werden.

Die Figur 7 zeigt eine vergrößerte Darstellung der Kanülenschürze 75 der Injektionsnadel 4 aus den Figuren 2 - 4. Die Kanülenschürze 75 weist einen Halteabschnitt 77 und einen daran anschließenden Verbindungsabschnitt 78 auf. Wie insbesondere aus der Figur 4 zu erkennen ist, verläuft durch die gesamte Kanülenschürze 75 eine Bohrung 89, in der die Kanüle 74 aufgenommen ist, wobei die Bohrung 89 zumindest zwei Abschnitte 89A, 89B mit unterschiedlichen Durchmessern aufweist. Der Abschnitt 89A ist zumindest größtenteils im Halteabschnitt 77, der Abschnitt 89B ist zumindest größtenteils im Verbindungsabschnitt 78 der Kanülenschürze 75 angeordnet. Selbstverständlich können auch mehr als zwei Abschnitte mit unterschiedlichen Durchmessern vorgesehen sein.

Der Innendurchmesser des Bohrungsabschnitts 89A ist zumindest in einem Abschnittsbereich etwa gleich dem Außendurchmesser der Kanüle 74, so dass die Kanüle 74 die Kanülenschürze 75 berührt und fest mit ihr verbunden, zum Beispiel verklebt oder vergossen, und in ihr gehalten ist. Der Durchmesser des Abschnitts 89B ist zumindest in einem Abschnittsbereich so bemessen, dass eine Ampulle 2 oder ein Teil einer Ampulle in diesen Abschnittsbereich einsetzbar ist.

Der Halteabschnitt 77 verläuft zu seinem freien, vorderen Ende hin konisch und weist in einem bevorzugten Ausführungsbeispiel an seinem anderen, der Verbindungsabschnitt 78 zugewandten Ende eine geometrische Ausformung 90, zum Beispiel eine Nut oder ein Polygon, zur Verbindung mit einem Nadelwechsler auf. An diese Ausformung 90 schließt ein ringförmiger, die Kanülenschürze 75 umgebender Flansch oder Wulst 91 an, der in einer bevorzugten Anwendung, wie weiter unten noch beschrieben wird, ein Rast- oder Eingriffselement 104 aufweist.

Der im Wesentlichen an seiner Außenseite zylindrisch geformte Verbindungsabschnitt 78 dient zur Verbindung der Kanülenschürze 75 mit einer Injektionsvorrichtung, insbesondere mit einem Handgriffelement 1, und mit einer Ampulle 2. An den Wulst 91 anschließend ist ein Kupplungselement, zum Beispiel eine Ringnut 83, als Teil einer Kupplungsvorrichtung vorgesehen, in die Rastelemente einer Injektionsvorrichtung zur Verbindung und Fixierung der Kanülenschürze 75 und damit einer Injektionsnadel an der Injektionsvorrichtung eingreifen können (siehe dazu auch Figur 3 und die zugehörige Beschreibung).

An die Ringnut 83 schließt ein Ringbund 92 an, der bevorzugt an der der Ringnut 83 abgewandten Seite eine Fase aufweist, so dass ein Rastelement, das in die Ringnut 83 einrasten soll, den Ringbund besser überwinden kann. Vom Ringbund 92 ausgehende erstrecken sich mehrere Funktionsteile, wobei zumindest einer dieser Funktionsteile als federndes Bauteil ausgebildet ist. Um die Federwirkung zu begünstigen, ist in einem bevorzugten Ausführungsbeispiel die Wandstärke zumindest des federnden Funktionsteils geringer als die Wandstärke des Ringbunds 92.

Ein erstes Funktionsteil ist als Spannelement 85, zum Beispiel als Flügel, als Teil einer Spannvorrichtung ausgebildet. Der Flügel 85 ist radial nach innen, in Richtung der Längsachse der Kanülenschürze 75 bewegbar und dient der Verspannung der Kanülenschürze 75 und der Injektionsnadel 4 mit der Ampulle 2 (siehe dazu auch Figur 4 und die dazugehörige Beschreibung). Ein anderes Funktionselement wird durch zumindest ein Kupplungselement 84, zum Beispiel in Form einer Kupplungsnase, gebildet, das Teil einer Kupplungs- oder Mitnehmervorrichtung zur Übertragung einer Drehbewegung und eines Drehmoments auf die Kanülenschürze 75 und die Injektionsnadel 4 ist (siehe dazu auch Figur 3 und die zugehörige Beschreibung).

Ein weiteres Funktionselement ist Teil einer unlösbaren Verriegelungsvorrichtung 76 zur Verriegelung der Ampulle 2 mit der Injektionsnadel 4. Das Funktionselement ist bevorzugt als federndes, längliches Rastelement 80 ausgebildet, auf dem besonders bevorzugt auch das Kupplungselement 84 angeordnet ist. Von dem zumindest einen Rastelement 80 steht radial nach Innen, in Richtung der Bohrung 89 eine Rastnase 81 mit einer Kontaktfläche 82 zum Eingriff in einen Rücksprung einer Ampulle 2 ab (siehe dazu detaillierte Beschreibung weiter unten).

In den Figuren 8 und 9 ist ein Injektionssystem 70 zur Injektion einer medizinischen Substanz in menschliches oder tierisches Gewebe, umfassend eine Ampulle 2 und eine Injektionsnadel 4, dargestellt, wobei in der Figur 8 die Ampulle 2 von der Injektionsnadel 4 getrennt ist und die Figur 9 die Ampulle 2 mit der Injektionsnadel 4 verbunden zeigt. Die in den beiden Figuren 8 und 9 dargestellte Injektionsnadel entspricht der bereits beschriebenen Injektionsnadel 4 mit der Kanülenschürze 75 der Figuren 2 - 4 und 7. Selbstverständlich ist es jedoch auch möglich das Injektionssystem 70 mit einer anders aufgebauten Injektionsnadel auszustatten, solange auch diese Injektionsnadel die für das Injektionssystem 70 wesentlichen technischen Merkmale aufweist.

Die Ampulle 2 entspricht einer herkömmlichen Ampulle und umfasst eine hohle Außenhülle 71 mit einem ersten und zweiten Ende, ein an einem Ende angeordnetes Septum 72 und eine am anderen Ende angeordnete, in die hohle Außenhülse 71 verschiebbare Verschlusskappe 73. Die Außenhülle 71 weist im Bereich jenes Endes, an dem das Septum 72 angeordnet ist, eine Verengung oder einen Rücksprung 79 auf. Das Septum 72 besteht aus einer scheibenförmige Gummiplatte, die an einem Ende der Außenhülle 71 angebracht und durch eine Metallmanschette 93 daran befestigt ist. Die Metallmanschette 93 weist eine Öffnung in ihrer Mitte auf, so dass die Kanüle 74 der Injektionsnadel 4 durch das Septum 72 in den Innenraum der Ampulle 2 eindringen kann.

Das Injektionssystem 70 weist eine unlösbare Verriegelungsvorrichtung 76 zur Verriegelung der Ampulle 2 mit der Injektionsnadel 4 auf, so dass nach dem Anschluss der Ampulle 2 an die Injektionsnadel 4 eine untrennbare Verbindung entsteht. Unter einer unlösbaren Verriegelungsvorrichtung wird jede Verriegelungsvorrichtung verstanden, die so ausgebildet ist, dass eine einmalig Verriegelung oder Verbindung zwischen der Ampulle 2 und der Injektionsnadel 4 herstellbar ist, wobei die Verriegelung zerstörungsfrei nicht mehr lösbar ist. Die Verriegelungsvorrichtung ist somit nicht dafür bestimmt und nicht entsprechend ausgebildet, dass sie wieder trennbar ist. Die Verbindung der Ampulle 2 mit der Injektionsnadel 4 ist somit für den Anwender untrennbar.

Bevorzugt ist die Verriegelungsvorrichtung 76 als unlösbare Rastvorrichtung aufgebaut, mit zumindest einem Rastelement 80 und zumindest einer Aufnahme, in die das Rastelement 80 eingreift, wobei besonders bevorzugt als Aufnahme die an der Ampulle 2 vorhandene Verengung oder der Rücksprung 79 verwendet wird, so dass hierfür keine eigene Aufnahme geschaffen werden muss. Dem gemäß ist das zumindest eine Rastelement 80 an der Injektionsnadel 4 angeordnet, bevorzugt am Verbindungsabschnitt 78 der Kanülenschürze 75, wie in Zusammenhang mit Figur 7 bereits erwähnt. Jedes Rastelement 80 umfasst eine federnd ausgebildete, längliche Lasche 80A mit einem freien Ende und einem gegenüber liegenden, mit dem Ringbund 92 verbundenen Ende. Die zumindest eine Lasche 80A ist durch zwei Schlitze 94 von angrenzenden Funktionselementen getrennt.

An ihrer zur Bohrung 89 gewandten Innenseite weist die Lasche 80A eine Rastnase 81 auf, die zum Eingriff in den Rücksprung 79 der Ampulle 2 vorgesehen ist. Die Rastnase 81 ist an ihrer, dem Halteabschnitt 77 zugewandten Seite mit einer Kontaktfläche 82 versehen, die über ihre gesamte Länge im Wesentlichen rechtwinkelig zum Verbindungsabschnitt 78 angeordnet ist. An der gegenüberliegenden Seite weist die Rastnase 81 eine Schräge oder Fase auf.

Beim Verbinden der Ampulle 2 mit der Injektionsnadel 4 wird die Ampulle 2 in die Bohrung 89 der Injektionsnadel 4 eingeschoben, bis die Rastnasen 81 mit ihren abgefasten Seiten die Metallmanschette 93 der Ampulle 2 berühren. Aufgrund der elastischen, federnden Ausführung der Laschen 80A biegen sich die Rastelemente 80 radial nach außen, so dass die Rastnasen 81 mit ihren abgefasten Seiten über die Metallmanschette 93 gleiten bis sie in den Rücksprung 79 der Ampulle 2 einrasten (siehe Figur 9). Der Aufbau der Rastnasen 81, insbesondere die rechtwinkelig zum Verbindungsabschnitt 78 ausgebildeten Kontaktflächen 82, die eine Gegenfläche des Rücksprungs 79 kontaktieren, und die Länge der Rastnasen 81, bewirken eine derart feste Verbindung, dass eine Trennung der Injektionsnadel 4 von der Ampulle 3, zum Beispiel durch axiales Auseinanderziehen der Ampulle 2 und der Injektionsnadel 4, nicht möglich ist. Die Ampulle 2 und die Injektionsnadel 4 bleiben damit nach ihrer Verbindung dauerhaft miteinander verriegelt, wodurch die Verletzungsgefahr für den Anwender verringert ist, da das Ende 74B der Kanüle 74 dauerhaft in der Ampulle 2 aufgenommen ist.

Aus der Figur 8 ist auch zu erkennen, dass die Injektionsnadel 4 ein weiteres Sicherheitsmerkmal zum Schutz des Anwenders vor ungewünschten Verletzungen aufweist. Die Kanüle 74 der Injektionsnadel 4 weist ein erstes Ende 74A zum Eindringen in menschliches oder tierisches Gewebe und ein zweites Ende 74B zum Durchstoßen des Septums 72 der Ampulle 2 auf, wobei die Kanülenschürze 75 die Kanüle 74 zumindest teilweise umgibt und wobei die Kanülenschürze 75 zum Schutz des Anwenders vor ungewollten Verletzungen über das zweite Ende 74B der Kanüle 74 ragt. Bevorzugt überragt die Kanülenschürze 75 die Kanüle 74 um wenige Millimeter, besonders bevorzugt um in etwa 2 - 5 mm. Durch diese Maßnahme wird vor allem die Gefahr des ungewollten Stechens beim Verbinden der Injektionsnadel 4 mit der Ampulle 2 oder einer Injektionsvorrichtung vermindert.

Die Figuren 8, 9 und 11 zeigen des Weiteren eine Injektionseinheit 100, die eine Injektionsnadel 4 mit einer Kanüle 74 und einer Kanülenschürze 75 und ein Schutzelement 101, das die Injektionsnadel 4 zumindest teilweise umgibt, umfasst. Zusätzlich ist eine Verbindungsvorrichtung 102, insbesondere eine Rastvorrichtung, mit zwei Fixpositionen 103A, 103B vorgesehen, wobei eine der beiden Fixpositionen als unlösbare Fixposition 103B ausgebildet ist und wobei die Injektionsnadel 4 und das Schutzelement 101 zur Einnahme der beiden Fixpositionen 103A, 103B verschiebbar zueinander ausgebildet sind.

Die dargestellte Injektionsnadel entspricht der bereits beschriebenen Injektionsnadel 4 mit der Kanülenschürze 75 der Figuren 2 - 4 und 7, es wird jedoch darauf verwiesen, dass es möglich ist, die Injektionseinheit 100 mit einer anders aufgebauten Injektionsnadel auszustatten, solange diese Injektionsnadel die für die Injektionseinheit 100 wesentlichen technischen Merkmale aufweist.

Das Schutzelement 101 ist ein längliches, mit einer Innenbohrung 109 versehenes, bevorzugt aus Kunststoff hergestelltes Element, in das zumindest ein Teil der Injektionsnadel 4 einfügbar ist. Die Innenbohrung 109 weist mehrere Abschnitte mit unterschiedlichen Innendurchmessern auf, so dass unterschiedlichen Abschnitte der Injektionsnadel 4, zum Beispiel der Halteabschnitt 77 und der Verbindungsabschnitt 78 in diesen Abschnitten aufgenommen werden können. Die Abschnitte mit den unterschiedlichen Innendurchmessern sind durch Ringschultern oder Stufen voneinander getrennt. Das Schutzelement 101 weist des Weiteren ein geschlossenes Ende 111 mit einer durchgehenden Querwand und ein offenes Ende mit einer Öffnung 110 zum Einschieben der Injektionsnadel 4 auf. Am Außenumfang des Schutzelements 101 sind Eingriffsmittel 107 für einen Nadelwechsler 108 vorgesehen, zum Beispiel ein oder mehrere Rücksprünge oder eine Nut. In einem bevorzugten Ausführungsbeispiel wird als Schutzelement 101 zumindest ein Teil der Verpackung der Injektionsnadel 4 verwendet, insbesondere jener Teil der Verpackung, der das mit der Schneidkante 96 versehene Ende der Kanüle 74 umgibt.

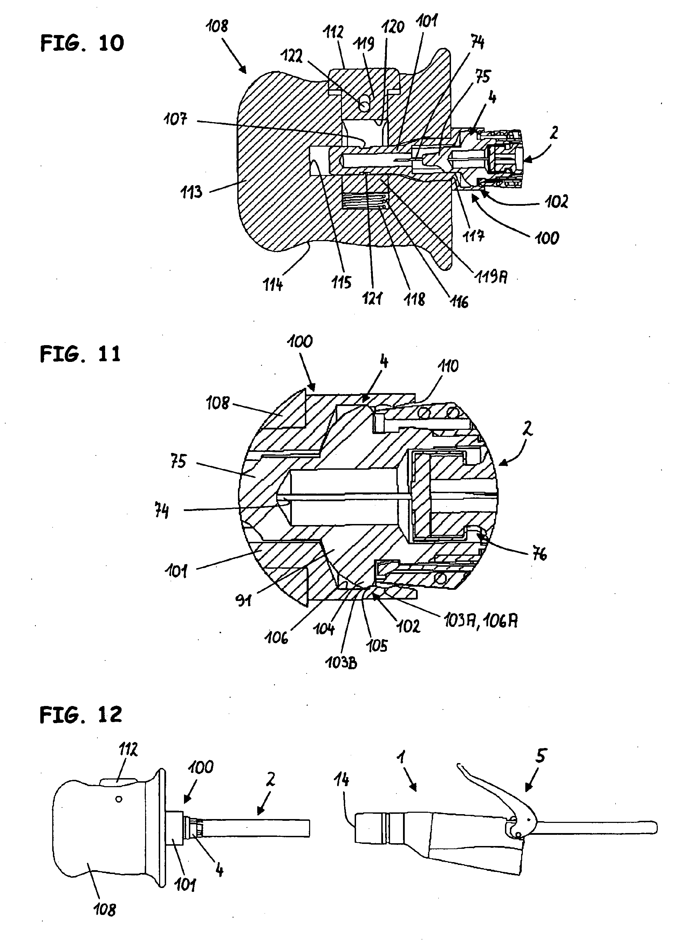

Die Verbindungsvorrichtung 102 umfasst Teile am Schutzelement 101 und Teile an der Injektionsnadel 4. An der Innenseite des Schutzelements 101 sind bevorzugt die Fixpositionen 103A, 103B, die insbesondere durch Rücksprünge oder Nuten 106A, 106B gebildet sind, vorgesehen, wo hingegen das Eingriffselement 104, insbesondere ein Rastelement, zum Eingreifen in die Fixpositionen 103A, 103B an der Injektionsnadel 4, insbesondere an der Kanülenschürze 75 vorgesehen ist (siehe Figur 11). Das Eingriffselement 104 wird durch den Flansch oder Wulst 91 bzw. durch einen Abschnitt davon, insbesondere durch eine Kante am Flansch 91 gebildet.

Die beiden Fixpositionen 103A, 103B sind derart neben- bzw. hintereinander angeordnet, dass das Eingriffselement 104 jeweils nur in eine der beiden Fixpositionen 103A, 103B eingreifen kann. Die Unlösbarkeit der Fixposition 103B wird in einem Ausführungsbeispiel durch eine im Wesentlichen rechtwinkelig angeordnete Kante 105 in Bezug auf die Innenwand der Bohrung 109 erzielt. Im Gegensatz dazu weist die andere, lösbar ausgebildete Fixposition 103A zumindest eine stetig ansteigende Seitenwand auf. Unter einer unlösbaren Fixposition wird jede Fixposition verstanden, die so ausgebildet ist, dass eine einmalig Verriegelung oder Verbindung zwischen der Injektionsnadel 4 und der unlösbaren Fixposition herstellbar ist, wobei die Verbindung zerstörungsfrei nicht mehr lösbar ist. Die Verbindung ist somit nicht dafür bestimmt und nicht entsprechend ausgebildet, dass sie wieder trennbar ist. Die Verbindung der Injektionsnadel 4 mit dem Schutzelement 101 ist somit für den Anwender untrennbar.

Die Verbindungsvorrichtung 102 ermöglicht, das Schutzelement 101 in einer doppelten Funktion zu verwenden, nämlich einerseits als Aufbewahrungs- und / oder Transport- und / oder Verpackungsbehälter, in dem zum Beispiel die sterile, unbenutzte Injektionsnadel 4 aufbewahrt ist, und andererseits als Schutzbehälter nach Abschluss der Verwendung der Injektionsnadel 4, wobei nach Abschluss der Verwendung sicher gestellt werden soll, dass die benützte Injektionsnadel 4 nicht versehentlich noch einmal benützt wird oder dass der Schutzbehälter sich von der benutzten Injektionsnadel löst und die Gefahr besteht, dass ein Anwender sich an der unbedeckten Kanülenspitze verletzt.

Dies wird durch die Verbindungsvorrichtung 102 mit den beiden Fixpositionen 103A, 103B erreicht. Dient das Schutzelement 101 der Aufbewahrung der Injektionsnadel 4, so befindet sich das Eingriffselement 104 in der lösbaren Fixposition 103A (siehe Figur 9), in die die Injektionsnadel 4 auch mehrmals gebracht und daraus wieder gelöst werden kann. Zur Verwendung der Injektionsnadel 4 wird diese aus dem Schutzelement 101 und der Verbindungsvorrichtung 102 durch die Öffnung 110 herausgezogen. Ist die Verwendung der Injektionsnadel 4 beendet und soll die Injektionsnadel 4 entsorgt werden, so wird das Eingriffselement 104 mit der unlösbaren Fixposition 103B in Eingriff gebracht. Dies erfolgt, in dem die Injektionsnadel 4 durch die Öffnung 110 in das Schutzelement 101 eingeschoben wird, wobei das Eingriffselement 104 zuerst in die zur Öffnung 110 näher gelegene, lösbare Fixposition 103A einrastet. Schiebt der Anwender die Injektionsnadel 4 weiter in die Bohrung 109 des Schutzelements 101 in Richtung des geschlossenen Endes 111, so gleitet das Eingriffselement 104 über die stetig ansteigende Seitenwand der lösbaren Fixposition 103A hinaus, überwindet die rechtwinkelig angeordnete Kante 105 und rastet in der unlösbaren Fixposition 103B ein (siehe Figur 11). Anschließend kann die Injektionsnadel 4 gemeinsam mit dem Schutzelement 101 entsorgt werden, ohne dass die Gefahr besteht, dass sich das Schutzelement 101 von der Injektionsnadel löst und Verletzungen verursacht.

In einem bevorzugten Ausführungsbeispiel bleibt die Injektionsnadel 4 mit der Ampulle 2 auch nach dem Einrasten in die unlösbaren Fixposition 103B verbunden, so dass beide Enden 74A, 74B der Kanüle 74 abgedeckt sind, wodurch das Verletzungsrisiko weiter verringert ist. Diese Situation ist in Figur 12 dargestellt, wobei, wie im Folgenden noch beschrieben ist, für die Handhabung des Schutzelements 101 ein Nadelwechsler 108 verwendet wird, so dass das Schutzelement 101 zum größten Teil im Nadelwechsler 108 aufgenommen ist. Die Injektionsnadel 4, die Ampulle 2 und das Schutzelement 101 sind zu einer Einheit verbunden am Nadelwechsler 108 befestigt und von der Injektionsvorrichtung, zum Beispiel einem medizinischen Handgriffelement 1, getrennt. Durch Betätigen des Druckknopfs 112 können die Injektionsnadel 4, die Ampulle 2 und das Schutzelement 101 vom Nadelwechsler 108 gelöst und entsorgt werden.

In Figur 10 ist ein Nadelwechsler 108 dargestellt, der das Entfernen und Einbringen der Injektionsnadel 4 in das Schutzelement 101 erleichtert. Der Nadelwechsler 108 umfasst einen Körper 113, der bevorzugt ergonomische geformt ist. Insbesondere weist der Nadelwechsler 108 an seinem Außenumfang eine oder mehrere Ausformungen 114, zum Beispiel Rücksprünge, Kerben, Nuten, Leisten oder Führungen, zum Eingriff und zum verbesserten Halt der Hand des Anwenders auf.

Im Körper 113 des Nadelwechslers 108 sind zwei gewinkelt, bevorzugt in etwa rechtwinkelig, zueinander angeordnete Bohrungen 115, 116 angeordnet. Die Bohrung 115 ist für die Aufnahme des Schutzelements 101 durch die Öffnung 117 vorgesehen und ist in ihrer Form der Außenform des Schutzelements 101 angepasst. Sie weist zum Beispiel mehrere Abschnitte mit unterschiedlichen Innendurchmessern auf. In der Bohrung 116 sind ein Federelement 118, insbesondere eine Spiralfeder, ein Schiebelemente 119 mit einer Bohrung 120, zum Beispiel ein verschiebbarer Schaft, und ein mit dem Schiebeelement 119 verbundener Druckknopf 112 oder ein anderes Betätigungselement vorgesehen. Der Druckknopf 112 ist in einer Ausnehmung der Bohrung 116 mit einem vergrößerten Innendurchmesser angeordnet und steht mit seinem freien Ende über die Bohrung 116 hinaus, so dass er für den Anwender gut zugänglich und einfach bedienbar ist. Das Schiebelement 119 wird durch einen Stift 122, der in einem Langloch des Schiebeelements 119 geführt wird, im Bohrerwechsler 108 fixiert.

Ist im Bohrerwechsler 108 kein Schutzelement 101 eingeführt, so drückt das Federelement 118 das Schiebeelement 119 in Richtung des Druckknopfs 112, so dass sich ein Teil 119A des Schiebeelements 119, der zwischen der Bohrung 120 und dem Federelement 118 angeordnet ist, in der Bohrung 115 befindet. Wird ein Schutzelement 101 in die Bohrung 115 eingeschoben, so verdrängt das Schutzelement 101 den Teil 119A gegen die Kraft des Federelements 118 in Richtung des Federelements 118, so dass zumindest ein Teil der Bohrung 120 des Schiebeelements 119 mit der Bohrung 115 in einer Linie ausgerichtet ist und das Schutzelement 101 durch die Bohrung 120 in Richtung des geschlossenen Endes der Bohrung 115 geschoben werden kann. Am Teil 119A des Schiebeelements 119 ist ein Rastelement 121, zum Beispiel eine Rastnase, vorgesehen, das in die Eingriffsmittel 107 des Schutzelements 101 einrastet. Durch das Rastelement 121 und die Federkraft des Federelements 118, das das Schutzelement 101 gegen die Innenwand der Bohrung 115 drückt, wird das Schutzelement 101 im Nadelwechsler fixiert, so dass die Injektionsnadel 4 aus dem Schutzelement 101 herausgenommen und darin eingefügt werden kann.

Das Lösen des Schutzelements 101 erfolgt durch Druck auf den Druckknopf 112, wodurch das Schiebeelement 119 gegen die Kraft des Federelements 118 in Richtung des Federelements 118 verschoben wird und das Schutzelement 101 freigibt. Wird das Schutzelement 101 als Verpackung und zur Entsorgung der Injektionsnadel 4 verwendet, so kann das Schutzelement 101 während der Verwendung der Injektionsnadel 4 im Nadelhalter 108 verbleiben.

Im Folgenden wird ein Verfahren zur Handhabung einer Injektionsnadel und zum Beund Entladen einer Injektionsvorrichtung, insbesondere eines medizinischen Handgriffelements 1 beschrieben. Das Verfahren ist so ausgestaltet, dass die Gefahr sich mit der Injektionsnadel 4 zu verletzen und unbeabsichtigt zu stechen für den Anwender erheblich reduziert ist. Das Verfahren besteht aus einer Vielzahl von Einzelschritten, von denen einige jeweils zu separaten Unterverfahren zusammengefasst werden können. Die Anwendung eines Einzelschritts oder eines oder mehrer dieser Unterverfahren, die zum Teil schon bei der Beschreibung der Figuren 1 - 12 dargestellt wurden, reduziert ebenfalls die Verletzungsgefahr für den Anwender, zumindest bei einem Teilschritt der Handhabung einer Injektionsnadel oder einer Injektionsvorrichtung. Der höchste Schutz in Form einer durchgehenden, geschlossenen Hygienekette wird jedoch bei Anwendung des im folgenden dargestellten Gesamtverfahrens erzielt.

Ausgangspunkt des Verfahrens ist eine unbenutzte, üblicherweise sterile Injektionsnadel, die in einer Verpackung, zum Beispiel einem Verpackungsbehälter aufgenommen ist. Bevorzugt ist die Injektionsnadel als Injektionsnadel 4 gemäß den Figuren 2 - 4 und 7 - 9 aufgebaut. Der Verpackungsbehälter ist zwei- oder mehrteilig aufgebaut und umfasst das Schutzelement 101. Die Injektionsnadel 4 ist im Schutzelement 101 aufgenommen und befindet sich in der lösbaren Fixposition 103A.

In einem ersten Schritt wird der Verpackungsbehälter geöffnet und alle Teile des Verpackungsbehälters außer dem Schutzelement 101 von der Injektionsnadel 104 entfernt. Diese Verpackungsteile können entsorgt werden, da sie im Weiteren nicht mehr benötigt werden. Dem Anwender steht somit eine Injektionseinheit 100, umfassend die Injektionsnadel 4 und das Schutzelement 101, zur Verfügung, wie sie in der Figur 8 abgebildet ist. Beide Enden 74A, 74B der Kanüle 74 sind durch das Schutzelement 101 bzw. durch die über das Kanülenende 74B ragende Kanülenschürze 75 abgedeckt, so dass der Anwender vor unbeabsichtigten Stichen durch die Kanüle geschützt ist.

Im nächsten Schritt verbindet der Anwender die Injektionseinheit 100 mit der Ampulle 2, in dem er die Ampulle 2 in die Kanülenschürze 75 einschiebt, wobei gleichzeitig die Kanüle 74 das Septum 72 durchsticht und in den mit der zu injizierenden Substanz gefüllten Innenraum der Ampulle 2 eindringt. Die Kanülenschürze 75 ist dabei so ausgebildet, dass die Ampulle beim Einschieben in Bezug auf die Kanüle zentriert wird. Bevorzugt ist zusätzlich eine unlösbare Verriegelungsvorrichtung 76 vorgesehen, so dass die Ampulle 2 und die Injektionsnadel 4 untrennbar miteinander verbunden sind. Die Situation nach dem Verbinden der Ampulle 2 mit der Injektionseinheit 100 ist in Figur 9 dargestellt. Wie im Folgenden noch zu erkennen ist, bleibt die Ampulle 2 während des gesamten restlichen Verfahrens einschließlich der Entsorgung mit der Injektionsnadel 4 verbunden, so dass das Kanülenende 74B durch die Ampulle 2 bedeckt ist und für den Anwender keine Gefahr besteht, sich mit diesem Kanülenende 74B zu verletzen.

Im nächsten Schritt wird die Injektionseinheit 100 gemeinsam mit der Ampulle 2 mit der Injektionsvorrichtung, insbesondere mit einem medizinischen Handgriffelement 1, verbunden. Die Verbindung erfolgt bei Verwendung eines medizinischen Handgriffelements 1 bevorzugt durch Einfügen der Ampulle 2 und der Injektionsnadel 4 durch eine Aufnahmeöffnung 14 an einem Ende des Handgriffelements 1, wie dies weiter oben schon beschrieben wurde. Durch die Verbindung mit einem medizinischen Handgriffelement 1 erfolgt bevorzugt gleichzeitig auch eine Kupplung zwischen der Ampulle 2 und / oder der Injektionsnadel 4 und einem Kupplungselement 10, 11 zur Übertragung einer Antriebsbewegung und eines Drehmoments und / oder eine Verspannung zwischen der Ampulle 2 und der Injektionsnadel 4 durch eine Spannvorrichtung und / oder eine Kopplung der Ampulle 2 mit der Injektionsnadel 4 zur drehfesten Verbindung der beiden Elemente durch eine Kopplungseinrichtung 9, wie es ebenfalls weiter oben schon ausführlich dargestellt wurde.

Als nächstes wird das Schutzelement 101 von der Injektionsnadel 4 entfernt, wobei dafür bevorzugt ein Nadelwechsler 108, wie bereits beschrieben, verwendet wird. Besonders bevorzugt verbleibt das Schutzelement 101 im Nadelwechsler 108. Die Injektionsvorrichtung ist nun für die Injektion der medizinischen Substanz einsatzbereit, dies ist exemplarisch in Figur 1 an einem medizinischen Handgriffelement 1 dargestellt.

Nach Abschluss der Verwendung der Injektionsnadel 4 wird diese in das Schutzelement 101 eingefügt, wobei, wie oben bereits beschrieben die Injektionsnadel 4 in die unlösbare Fixposition 103B verschoben wird, so dass eine unlösbare Verbindung zwischen der Injektionsnadel 4 und dem Schutzelement 101 entsteht (siehe Figur 11). Wie bereits erwähnt ist bevorzugt das Schutzelement 101 Teil der Verpackung der Injektionsnadel und besonders bevorzugt im Nadelwechsler 108 eingespannt, es ist jedoch auch möglich ein eigenes, nicht als Verpackung für die unbenutzte Injektionsnadel 4 verwendetes Schutzelement 101 zu verwenden bzw. das Einfügen der Injektionsnadel 4 in das Schutzelement 101 ohne Nadelwechsler 108 durchzuführen.

Im nächsten Schritt werden die Ampulle 2, die Injektionsnadel 4 und das Schutzelement 101 von der Injektionsvorrichtung, insbesondere von dem medizinischen Handstück 1, getrennt, wie dies in Figur 12 dargestellt ist.

Abschließend werden die Ampulle 2, die Injektionsnadel 4 und das Schutzelement 101 als eine Einheit, und bevorzugt durch die Verriegelungsvorrichtung 76 und durch die Verbindungsvorrichtung 102 unlösbar aneinander gebunden, gemeinsam entsorgt.

Wie aus der Beschreibung des Verfahrens und des Aufbaus der Injektionsnadel 4, des Injektionssystems 70 und der Injektionseinheit 100 hervorgeht, sind beide Enden 74A, 74B der Kanüle 74 während des gesamten Verfahrens - mit Ausnahme der Verwendung der Nadel unmittelbar vor, während und unmittelbar nach der Injektion - abgedeckt, wodurch dem Anwender ein maximaler Schutz vor Verletzungen geboten wird.

Die Erfindung ist nicht auf die beschriebenen Ausführungsbeispiele beschränkt, sondern umfasst alle Ausführungen, die das prinzipielle, sinngemäße Funktionsprinzip der Erfindung anwenden oder beinhalten. So sind insbesondere die beschriebenen Injektionssysteme, Injektionseinheiten, Injektionsnadeln und die Handhabungsverfahren dafür nicht nur in Verbindung mit dem beschriebenen medizinischen Handgriffelement verwendbar, sondern auch mit allen anderen Injektionsvorrichtungen, zum Beispiel als Teil von Spritzen oder mit Spritzengestellen.

Auch sind alle unterschiedlichen beschriebenen Ausführungsbeispiele, soweit nicht bereits explizit darauf hingewiesen wurde, miteinander kombinierbar, mehrere oder alle der dargestellten Verfahren als Teil eines Gesamtverfahrens hintereinander oder gleichzeitig ausführbar, die beschriebenen Vorrichtungen in diesen Verfahren oder in einzelnen Verfahrensschritten verwendbar bzw. die Verfahren mit den dargestellten Vorrichtungen durchführbar.

## Patentansprüche

1. Medizinisches Handgriffelement (1) zur Injektion einer medizinischen Substanz aus einer Ampulle (2) in menschliches oder tierisches Gewebe mit:
einer Ampulle (2) und einer Injektionsnadel (4, 4'),
einer Antriebsvorrichtung (3) zum rotierenden Antrieb der Ampulle (2) und der Injektionsnadel (4, 4'),
einer Fördervorrichtung (5) zur Förderung der medizinischen Substanz aus der Ampulle (2) und
einer Kopplungseinrichtung (9) zur drehfesten Verbindung der Ampulle (2) mit der Injektionsnadel (4, 4'), wobei die Kopplungseinrichtung (9) zumindest ein Kupplungselement einer Kupplungsvorrichtung zur Übertragung des Drehmoments von der Antriebsvorrichtung (3) auf die Ampulle (2) und die Injektionsnadel (4, 4') aufweist, wobei die Kopplungseinrichtung (9) eine Anschlussvorrichtung (6, 6') für die Ampulle (2) und die Injektionsnadel (4, 4') aufweist, die eine rotierbare Hohlwelle (7, 7') umfasst, und wobei die Kopplungseinrichtung (9) ein erstes Kupplungselement (8) zur Drehmomentübertragung auf die Injektionsnadel (4, 4') und ein zweites Kupplungselement (8', 8") zur Drehmomentübertragung auf die Ampulle (2) aufweist, das an der Hohlwelle (7, 7') vorgesehen ist, **dadurch gekennzeichnet, dass**
die Kopplungseinrichtung (9) dazu ausgebildet ist, mit der Ampulle (2) und der Injektionsnadel (4, 4') eine Einheit zu bilden, auf die im Wesentlichen dasselbe Drehmoment einwirkt und die im Wesentlichen mit derselben Drehzahl rotierbar ist, so dass die Kopplungseinrichtung (9) eine Verdrehsicherung zur Vermeidung einer Relativbewegung zwischen der Ampulle (2) und der Injektionsnadel (4, 4') bildet.

2. Medizinisches Handgriffelement (1) nach Anspruch 1, **dadurch gekennzeichnet, dass** das Kupplungselement (8, 8', 8") zumindest eine Federlasche (11), einen Federarm, eine Spannfläche, ein Gewinde (10), einen Mitnehmer oder ein Formschlusselement umfasst.

3. Medizinisches Handgriffelement (1) nach einem der vorhergehenden Ansprüche, ' **dadurch gekennzeichnet, dass**
an der Kopplungseinrichtung (9) eine Spannvorrichtung (12) zum Verspannen der Ampulle (2) mit der Injektionsnadel (4, 4') vorgesehen ist.

4. Medizinisches Handgriffelement (1) nach Anspruch 3, **dadurch gekennzeichnet, dass** die Spannvorrichtung (12) zum Verspannen der Ampulle (2) mit der Injektionsnadel (4, 4') an einem Kupplungselement (8') vorgesehen oder als Teil davon ausgebildet ist.

5. Medizinisches Handgriffelement (1) nach Anspruch 3 oder 4, **dadurch gekennzeichnet, dass**
die Spannvorrichtung (12) zumindest eine konische Fläche (13) aufweist.

6. Medizinisches Handgriffelement (1) nach einem der vorhergehenden Ansprüche, **gekennzeichnet durch**
eine Aufnahmeöffnung (14) für die Ampulle (2), die an einem Ende des Handgriffelements (1) angeordnet ist und die derart ausgebildet ist, dass die Ampulle (2) von diesem Ende des Handgriffelements (1) **durch** die Aufnahmeöffnung (14) in das Handgriff element (1) einfügbar ist.

7. Medizinisches Handgriffelement (1) nach Anspruch 6, **dadurch gekennzeichnet, dass** die Aufnahmeöffnung (14) als Teil der Anschlussvorrichtung (6, 6') ausgebildet ist.

8. Medizinisches Handgriffelement (1) nach einem der vorhergehenden Ansprüche,
**dadurch gekennzeichnet, dass**
die Fördervorrichtung (5) an ihrem der Ampulle (2) zugewandten Ende ein drehbares Schubteil (34) aufweist, um ein Abbremsen der in Rotation versetzbaren Ampulle (2) durch die Fördervorrichtung (5) zu unterbinden.

9. Medizinisches Handgriffelement (1) nach Anspruch 8, **dadurch gekennzeichnet, dass**
das Schubteil (34) einen Keil (35) umfasst, der auf einer Welle (36) befestigt ist, die drehbar mit einem Lager (37) verbunden ist.

10. Medizinisches Handgriffelement (1) nach einem der vorhergehenden Ansprüche, **dadurch gekennzeichnet, dass**
das erste Kupplungselement (8) zur Drehmomentübertragung auf die Injektionsnadel (4, 4') an der Hohlwelle (7, 7') vorgesehen ist.

11. Medizinisches Handgriffelement (1) nach Anspruch 10, **dadurch gekennzeichnet, dass**
das erste Kupplungselement (8) zur Drehmomentübertragung auf die Injektionsnadel (4, 4') mehrere Mitnehmerelemente (46), insbesondere Aufnahmen oder Rücksprünge, zum Eingriff von Kupplungselementen (84) der Injektionsnadel (4, 4') umfasst.

12. Medizinisches Handgriffelement (1) nach Anspruch 11, **dadurch gekennzeichnet, dass**
die Mitnehmerelemente. (46) durch Trennelemente (47), zum Beispiel Stege, voneinander getrennt sind.

13. Medizinisches Handgriffelement (1) nach Anspruch 10, **dadurch gekennzeichnet, dass**
das erste Kupplungselement (8) zur Drehmomentübertragung auf die Injektionsnadel (4, 4') einen Abschnitt (7A) mit einem verbreitertem Durchmesser an einem Ende der Hohlwelle (7, 7') aufweist, an dem eine Bajonettverbindungen, eine formschlüssige Verbindung oder ein Innengewinde (10A) vorgesehen ist, das mit einem Außengewinde (10B) der Injektionsnadel (4, 4') verschraubbar ist.

14. Medizinisches Handgriffelement (1) nach Anspruch 10, **dadurch gekennzeichnet, dass**
das erste Kupplungselement (8) zur Drehmomentübertragung auf die Injektionsnadel (4, 4') einen mit der Hohlwelle (7, 7') verbundenen Fortsatz (49, 50) aufweist, der mit einem Außengewinde (48B) zur Verbindung mit der Injektionsnadel (4, 4') versehen ist.

15. Medizinisches Handgriffelement (1) nach Anspruch 14, **dadurch gekennzeichnet, dass**
der Fortsatz (49, 50) und der daran anschließende Wandabschnitt der Hohlwelle (7, 7') eine schmale Bohrung (51) aufweisen, durch die die Kanüle (74, 74') der Injektionsnadel (4, 4') führbar ist.

16. Medizinisches Handgriffelement (1) nach einem der vorhergehenden Ansprüche, **dadurch gekennzeichnet, dass**
zur axialen Fixierung der Injektionsnadel (4, 4') am Handgriffelement (1) eine Spannhülse (40) mit mehreren Federarmen (41) zum Eingriff in eine Ringnut (83) der Kanülenschürze (75) der Injektionsnadel (4, 4') vorgesehen ist.

17. Medizinisches Handgriffelement (1) nach Anspruch 16, **dadurch gekennzeichnet, dass**
die Federarme (41) von einem Stellelement, zum Beispiel einer Schiebehülse (42), umgeben sind, das entlang der Längsachse des Handgriffelements (1) verschiebbar ist und das eine radiale Bewegung der Federarm (41) in die Ringnut (83) und aus der Ringnut (83) bewirkt.

18. Medizinisches Handgriffelement (1) nach einem der vorhergehenden Ansprüche, **dadurch gekennzeichnet, dass**
das zweite Kupplungselement (8', 8") mehrere Spannlaschen (11, 53) umfasst, die nach innen, in Richtung der Längsachse des Handgriffelements (1) vorgespannt sind.

19. Medizinisches Handgriffelement (1) nach einem der vorhergehenden Ansprüche, **dadurch gekennzeichnet, dass**
das zweite Kupplungselement (8', 8") an einem Ende der Hohlwelle (7, 7') angeordnet ist.

20. Medizinisches Handgriffelement (1) nach einem der vorhergehenden Ansprüche, **dadurch gekennzeichnet, dass**
das Handgriffelement (1) zweiteilig ausgebildet ist, wobei die Hohlwelle (7, 7') nicht teilbar ausgebildet ist.

21. Medizinisches Handgriffelement (1) nach einem der Ansprüche 1 - 19, **dadurch gekennzeichnet, dass**
das Handgriffelement (1) und die Hohlwelle (7, 7') zweiteilig ausgebildet sind, wobei in getrenntem Zustand jeweils ein Hohlwellenabschnitt (7'A, 7'B) in jeweils einem Teil (15A, 15B) des Handgriffelements (1) verbleibt.

22. Medizinisches Handgriffelement (1) nach einem der vorhergehenden Ansprüche, **dadurch gekennzeichnet, dass**
die Antriebsvorrichtung (3) in einer Verbreiterung der Außenhülse (15) angeordnet ist, die in Richtung des Kopfabschnitts (15A) des Handgriff elements (1) abnimmt.

23. Medizinisches Handgriffelement (1) nach einem der vorhergehenden Ansprüche, **dadurch gekennzeichnet, dass**
die Antriebsvorrichtung (3) schräg, in einem spitzen Winkel zur Längsachse des Handgriff elements (1) angeordnet ist.

24. Medizinisches Handgriffelement (1) nach einem der vorhergehenden Ansprüche, **dadurch gekennzeichnet, dass**
an der Außenseite der Hohlwelle (7, 7') ist ein Zahnrad (19) vorgesehen ist, das Teil der Antriebsvorrichtung (3) ist.

25. Medizinisches Handgriffelement (1) nach einem der vorhergehenden Ansprüche, **gekennzeichnet durch**
eine Ampulle (2) mit einer hohlen Außenhülle (71) mit einem ersten und zweiten Ende, einem an einem Ende angeordneten Septum (72) und einer am anderen Ende angeordneten, in die hohle Außenhülse (71) verschiebbaren Verschlusskappe (73), wobei das Septum (72) aus einer scheibenförmige Gummiplatte besteht.

## Claims

1. A medical hand-held element (1) for injecting a medical substance from an ampoule (2) into human or animal tissue, comprising:
an ampoule (2) and an injection needle (4, 4'),
a drive device (3) for the rotating drive of the ampoule (2) and the injection needle (4, 4')
a delivery device (5) for delivering the medical substance from the ampoule (2) and
a coupling unit (9) for a rotationally fixed connection of the ampoule (2) to the injection needle (4, 4'), wherein the coupling unit (9) has at least one coupling element of a coupling device for transfer of the torque from the drive device (3) to the ampoule (2) and the injection needle (4, 4'), wherein the coupling unit (9) has a connecting device (6, 6') for the ampoule (2) and the injection needle (4, 4'), comprising a rotatable hollow shaft (7, 7'), and wherein the coupling unit (9) has a first coupling element (8) for transfer of the torque to the injection needle (4, 4') and a second coupling element (8', 8") for transfer of the torque to the ampoule (2), which is provided on the hollow shaft (7, 7'), **characterized in that**
the coupling unit (9) is configured to form a unit with the ampoule (2) and the injection needle (4, 4') upon which substantially the same torque acts and which is rotatable at substantially the same rotational speed, so that the coupling unit (9) forms a twist-proof device to prevent a relative movement between the ampoule (2) and the injection needle (4, 4').

2. The medical hand-held element (1) according to Claim 1, **characterized in that**
the coupling element (8, 8', 8") comprises at least one spring shackle (11), a spring arm, a clamping surface, a thread (10), a driver or a form-fitting element.

3. The medical hand-held element (1) according to any one of the preceding claims, **characterized in that**
a clamping device (12) is provided on the coupling unit (9) for clamping the ampoule (2) to the injection needle (4, 4').

4. The medical hand-held element (1) according to Claim 3, **characterized in that**
the clamping device (12) for clamping the ampoule (2) to the injection needle (4, 4') is provided on a coupling element (8') or is designed as part of same.

5. The medical hand-held element (1) according to Claim 3 or 4, **characterized in that**
the clamping device (12) has at least one conical surface (13).

6. The medical hand-held element (1) according to any one of the preceding claims, **characterized by**
a receiving opening (14) for the ampoule (2), which is arranged at one end of the hand-held element (1) and which is designed so that the ampoule (2) can be inserted from this end of the hand-held element (1) through the receiving opening (14) into the hand-held element (1).

7. The medical hand-held element (1) according to Claim 6, **characterized in that**
the receiving opening (14) is designed as part of the connecting device (6, 6').

8. The medical hand-held element (1) according to any one of the preceding claims, **characterized in that**
the delivery device (5) has a rotatable push part (34) on its end facing the ampoule (2), to avoid a braking of the ampoule (2) which can be induced to rotate by the delivery device (5).

9. The medical hand-held element (1) according to Claim 8, **characterized in that**
the push part (34) comprises a wedge (35), which is attached to a shaft (36) that is rotatably connected to a bearing (37).

10. The medical hand-held element (1) according to any one of the preceding claims, **characterized in that**
the first coupling element (8) for transfer of the torque to the injection needle (4, 4') is provided on the hollow shaft (7, 7').

11. The medical hand-held element (1) according to Claim 10, **characterized in that**
the first coupling element (8) for transfer of the torque to the injection needle (4, 4') comprises several driver elements (46), in particular receptacles or recesses, for engagement of coupling elements (84) of the injection needles (4, 4').

12. The medical hand-held element (1) according to Claim 11, **characterized in that**
the driver elements (46) are separated from one another by separation elements (47), for example, webs.

13. The medical hand-held element (1) according to Claim 10, **characterized in that**
the first coupling element (8) for transfer of the torque to the injection needle (4, 4') has a section (7A) with a wider diameter at one end of the hollow shaft (7, 7'), on which a bayonet connection, a form-fitting connection or an inside thread (10A) is provided, which can be screwed onto an outside thread (10B) of the injection needle (4, 4').

14. The medical hand-held element (1) according to Claim 10, **characterized in that**
the first coupling element (8) for transfer of the torque to the injection needle (4, 4') has a protrusion (49, 50) connected to the hollow shaft (7, 7'), said protrusion being provided with an outside thread (48B) for connection to the injection needle (4, 4').

15. The medical hand-held element (1) according to Claim 14, **characterized in that**
the protrusion (49, 50) and the wall section of the hollow shaft (7, 7') connected thereto have a narrow bore (51), through which the cannula (74, 74') of the injection needle (4, 4') can be guided.

16. The medical hand-held element (1) according to any one of the preceding claims, **characterized in that**
a clamping sleeve (40) having several spring arms (41) for engaging in an annular groove (83) of the cannula skirt (75) of the injection needle (4, 4') is provided for axial fixation of the injection needle (4, 4') on the hand-held element (1).

17. The medical hand-held element (1) according to Claim 16, **characterized in that**
the spring arms (41) are surrounded by a control element, for example, a sliding sleeve (42), which can be displaced along the longitudinal axis of the hand-held element (1) and which causes a radial movement of the spring arm (41) into the annular groove (83) and out of the annular groove (83).

18. The medical hand-held element (1) according to any one of the preceding claims, **characterized in that**
the second coupling element (8', 8") comprises several tension straps (11, 53), which are prestressed toward the inside, in the direction of the longitudinal axis of the hand-held element (1).

19. The medical hand-held element (1) according to any one of the preceding claims, **characterized in that**
the second coupling element (8', 8") is arranged at an end of the hollow shaft (7, 7').

20. The medical hand-held element (1) according to any one of the preceding claims, **characterized in that**
the hand-held element (1) is designed in two parts, wherein the hollow shaft (7, 7') is designed to be non-divisible.

21. The medical hand-held element (1) according to any one of Claims 1 - 19, **characterized in that**
each of the hand-held element (1) and the hollow shaft (7, 7') are designed in two parts, wherein in a separated state one hollow shaft section (7'A, 7'B) remains in each part (15A, 15B) of the hand-held element (1).

22. The medical hand-held element (1) according to any one of the preceding claims, **characterized in that**
the drive device (3) is arranged in a widened area of the outer sleeve (15), decreasing in the direction of the head section (15A) of the hand-held element (1).

23. The medical hand-held element (1) according to any one of the preceding claims, **characterized in that**
the drive device (3) is arranged in a sloping manner, at an acute angle to the longitudinal axis of the hand-held element (1).

24. The medical hand-held element (1) according to any one of the preceding claims, **characterized in that**
a gearwheel (19), which is part of the drive device (3), is provided on the outside of the hollow shaft (7, 7').

25. The medical hand-held element (1) according to any one of the preceding claims, **characterized by**
an ampoule (2) having a hollow outer shell (71) with a first and a second end, a septum (72) arranged at one end and a closing cap (73) which is arranged on the other end and is displaceable into the hollow outer sleeve (71), wherein the septum (72) consists of a disk-shaped rubber panel.

## Revendications

1. Elément à poignée médical (1) destiné à l'injection d'une substance médicale d'une ampoule (2) dans un tissu humain ou animal comprenant :
une ampoule (2) et une aiguille d'injection (4, 4'),
un dispositif d'entraînement (3) pour l'entraînement en rotation de l'ampoule (2) et de l'aiguille d'injection (4, 4'),
un dispositif de transport (5) pour le transport de la substance médicale de l'ampoule (2) et
un dispositif de couplage (9) pour la liaison résistante à la torsion de l'ampoule (2) avec l'aiguille d'injection (4, 4'), sachant que le dispositif de couplage (9) présente au moins un élément d'accouplement d'un dispositif d'accouplement pour transmettre le couple de rotation du dispositif d'entraînement (3) à l'ampoule (2) et à l'aiguille d'injection (4, 4'), sachant que le dispositif de couplage (9) présente un dispositif de liaison (6, 6') pour l'ampoule (2) et l'aiguille d'injection (4, 4'), qui comprend un arbre creux rotatif (7, 7') et sachant que le dispositif de couplage (9) présente un premier élément d'accouplement (8) pour la transmission du couple de rotation à l'aiguille d'injection (4, 4') et un deuxième élément d'accouplement (8', 8") pour la transmission du couple de rotation à l'ampoule (2) qui est prévu sur l'arbre creux (7, 7'), **caractérisé en ce que**,
le dispositif de couplage (9) est conçu pour former avec l'ampoule (2) et l'aiguille d'injection (4, 4') une unité sur laquelle agit sensiblement le même couple de rotation et qui peut tourner avec sensiblement la même vitesse, de façon à ce que le dispositif de couplage (9) forme une sécurité anti-torsion pour éviter un mouvement relatif entre l'ampoule (2) et l'aiguille d'injection (4, 4').

2. Elément à poignée médical (1) selon la revendication 1, **caractérisé en ce que** l'élément d'accouplement (8, 8', 8") comprend au moins une lame-ressort (11), un bras de ressort, une surface de serrage, un filetage (10), un entraîneur ou un élément de complémentarité de forme.

3. Elément à poignée médical (1) selon l'une des revendications précédentes, **caractérisé en ce**
**qu'**un dispositif de serrage (12) est prévu sur le dispositif de couplage (9) pour serrer l'ampoule (2) avec l'aiguille d'injection (4, 4').

4. Elément à poignée médical (1) selon la revendication 3, **caractérisé en ce que**
l'élément de serrage (12) pour serrer l'ampoule (2) avec l'aiguille d'injection (4, 4') est prévu sur un élément d'accouplement (8') ou est formé comme une partie de celui-ci.

5. Elément à poignée médical (1) selon la revendication 3 ou 4, **caractérisé en ce que**
le dispositif de serrage (12) présente au moins une face conique (13).

6. Elément à poignée médical (1) selon l'une des revendications précédentes, **caractérisé par**
une ouverture de réception (14) pour l'ampoule (2), qui est disposée sur une extrémité de l'élément à poignée (1) et qui est ainsi formée que l'ampoule (2) peut être intégrée dans l'élément à poignée (1) à travers l'ouverture de réception (14), via cette extrémité de l'élément à poignée (1).

7. Elément à poignée médical (1) selon la revendication 6, **caractérisé en ce que**
l'ouverture de réception (14) est formée comme une partie du dispositif de liaison (6, 6').

8. Elément à poignée médical (1) selon l'une des revendications précédentes, **caractérisé en ce que**,
le dispositif de transport (5) présente sur son extrémité tournée vers l'ampoule (2), une pièce coulissante rotative (34) pour empêcher un ralentissement de l'ampoule (2) pouvant être mise en rotation, par le dispositif de transport (5).

9. Elément à poignée médical (1) selon la revendication 8, **caractérisé en ce que**
la pièce coulissante (34) présente un taquet (35) qui est fixé sur un arbre (36), lequel est relié en rotation avec un palier (37).

10. Elément à poignée médical (1) selon l'une des revendications précédentes, **caractérisé en ce que**
le premier élément d'accouplement (8) pour la transmission du couple de rotation à l'aiguille d'injection (4, 4') est prévu sur l'arbre creux (7, 7').

11. Elément à poignée médical (1) selon la revendication 10, **caractérisé en ce que**
le premier élément d'accouplement (8) pour la transmission du couple de rotation à l'aiguille d'injection (4, 4') présente plusieurs éléments entraîneurs (46), en particulier des réceptions ou des retraits, pour mettre en prise des éléments d'accouplement (84) de l'aiguille d'injection (4, 4').

12. Elément à poignée médical (1) selon la revendication 11, **caractérisé en ce que**
les éléments entraîneurs (46) sont séparés les uns des autres par des éléments séparateurs (47), par exemple des traverses.

13. Elément à poignée médical (1) selon la revendication 10, **caractérisé en ce que**
le premier élément d'accouplement (8) pour la transmission du couple de rotation à l'aiguille d'injection (4, 4') présente un segment (7A) avec un diamètre élargi sur une extrémité de l'arbre creux (7, 7'), sur lequel une liaison à baïonnette, une liaison par complémentarité de forme ou un filetage femelle (10A) est prévu, lequel peut être vissé à un filetage mâle (10B) de l'aiguille d'injection (4, 4').

14. Elément à poignée médical (1) selon la revendication 10, **caractérisé en ce que**
le premier élément d'accouplement (8) pour la transmission du couple de rotation à l'aiguille d'injection (4, 4') présente un appendice (49, 50) relié à l'arbre creux (7, 7') qui est muni d'un filetage mâle (48B) pour être relié à l'aiguille d'injection (4, 4').

15. Elément à poignée médical (1) selon la revendication 14, **caractérisé en ce que**
l'appendice (49, 50) et la section de paroi de l'arbre creux (7, 7') qui se raccorde dessus présentent un alésage fin (51) à travers lequel la canule (74, 74') de l'aiguille d'injection (4, 4') peut être introduite.

16. Elément à poignée médical (1) selon l'une des revendications précédentes, **caractérisé en ce que**
pour la fixation axiale de l'aiguille d'injection (4, 4') sur l'élément à poignée (1), un manchon de serrage (40) avec plusieurs bras de ressort (41) est prévu pour la mise en prise dans une gorge annulaire (83) du tablier de canule (75) de l'aiguille d'injection (4, 4').

17. Elément à poignée médical (1) selon la revendication 16, **caractérisé en ce que**
les bras de ressort (41) sont entourés par un élément de réglage, par exemple un manchon coulissant (42), qui peut être poussé le long de l'axe longitudinal de l'élément à poignée (1) et qui provoque un mouvement radial du bras de ressort (41) dans la gorge annulaire (83) et hors de la gorge annulaire (83).

18. Elément à poignée médical (1) selon l'une des revendications précédentes, **caractérisé en ce que**
le deuxième élément d'accouplement (8', 8") comprend plusieurs languettes de serrage (11, 53) qui sont précontraintes vers l'intérieur, en direction de l'axe longitudinal de l'élément à poignée (1).

19. Elément à poignée médical (1) selon l'une des revendications précédentes, **caractérisé en ce que**
le deuxième élément d'accouplement (8', 8") est disposé sur une extrémité de l'arbre creux (7, 7').

20. Elément à poignée médical (1) selon l'une des revendications précédentes, **caractérisé en ce que**
l'élément à poignée (1) est formé de deux parties, sachant que l'arbre creux (7, 7') n'est pas séparable.

21. Elément à poignée médical (1) selon l'une des revendications 1 à 19, **caractérisé en ce que**
l'élément à poignée (1) et l'arbre creux (7, 7') sont en deux parties, sachant que dans l'état séparé, respectivement une section de l'arbre creux (7'A, 7'B) reste dans respectivement une partie (15A, 15B) de l'élément à poignée (1).

22. Elément à poignée médical (1) selon l'une des revendications précédentes, **caractérisé en ce que**
le dispositif d'entraînement (3) est disposé dans un élargissement du manchon extérieur (15) qui rétrécit en direction de la section de tête (15A) de l'élément à poignée (1).

23. Elément à poignée médical (1) selon l'une des revendications précédentes, **caractérisé en ce que**
le dispositif d'entraînement (3) est disposé en biais, dans un angle aigu par rapport à l'axe longitudinal de l'élément à poignée (1).

24. Elément à poignée médical (1) selon l'une des revendications précédentes, **caractérisé en ce que**
sur le côté extérieur de l'arbre creux (7, 7'), une roue dentée (19) est prévue qui fait partie du dispositif d'entraînement (3).

25. Elément à poignée médical (1) selon l'une des revendications précédentes, **caractérisé par**
une ampoule (2) comprenant une enveloppe extérieure creuse (71) avec une première et une deuxième extrémité, un septum (72) disposé sur une extrémité et un capuchon (73) pouvant être poussé dans l'enveloppe extérieure creuse (71), disposé sur l'autre extrémité, sachant que le septum (72) est composé d'une plaque de caoutchouc en forme de disque.
